(19) Europäisches Patentamt
European Patent Office
Office européen des brevets

(11) EP 1 847 247 B1

(12) # FASCICULE DE BREVET EUROPEEN

(45) Date de publication et mention de la délivrance du brevet:
**20.05.2015 Bulletin 2015/21**

(51) Int Cl.:
***A61K 8/34*** *(2006.01)* ***A61Q 19/08*** *(2006.01)*
***A61Q 19/00*** *(2006.01)* ***A61Q 19/02*** *(2006.01)*
***A61K 31/05*** *(2006.01)*

(21) Numéro de dépôt: **07105887.9**

(22) Date de dépôt: **10.04.2007**

(54) **Compositions comprenant un dérivé diphényl-méthane hydroxylé**

Zusammensetzung mit einem hydroxylierten Diphenylmethan-derivat

Compositions comprising a hydroxylated diphenyl-methane derivative

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **21.04.2006 FR 0651417**

(43) Date de publication de la demande:
**24.10.2007 Bulletin 2007/43**

(73) Titulaire: **L'Oréal**
**75008 Paris (FR)**

(72) Inventeur: **Baldo, Francine**
**92330 SCEAUX (FR)**

(74) Mandataire: **Brohmi, Karim**
**L'OREAL**
**D.I.P.I.**
**25-29 Quai Aulagnier**
**92665 Asnières-sur-Seine Cedex (FR)**

(56) Documents cités:

| | |
|---|---|
| **EP-A- 1 172 077** | **EP-A- 1 493 774** |
| **EP-A- 1 566 171** | **EP-A- 1 598 058** |
| **EP-B1- 0 975 323** | **WO-A-03/059314** |
| **WO-A-2004/105736** | **DE-A- 10 324 567** |
| **FR-A- 2 761 959** | **US-A- 5 399 785** |

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 847 247 B1

## Description

La présente invention se rapporte à des compositions, notamment cosmétiques, contenant au moins un dérivé diphényl-méthane hydroxylé de formule (I)

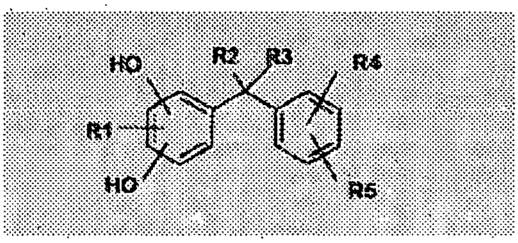

Il est connu d'utiliser des actifs dans des compositions cosmétiques et/ou dermatologiques, par exemple en vue de soigner ou de traiter ou d'apporter des effets bénéfiques à la peau. Toutefois, certains de ces actifs présentent l'inconvénient d'être instables dans les solvants cosmétiques classiques et/ou de se dégrader facilement, notamment au contact de l'eau, en particulier à cause de phénomènes d'oxydation. Ils perdent ainsi rapidement leur activité au cours du temps et cette instabilité va à l'encontre de l'efficacité recherchée.

Les dérivés diphényl-méthane hydroxylés sont connus de la demande WO2004/105736 dans des compositions sous forme d'émulsion. Ces dérivés diphényl-méthane hydroxylés sont décrits dans cette demande comme inhibiteurs de tyrosinase, utilisables notamment dans des compositions dépigmentantes.

Ces dérivés diphényl-méthane hydroxylés, du fait notamment de leur structure aromatique et de leur caractère lipophile, présentent l'inconvénient d'être instables et/ou faiblement solubles dans les solvants classiques utilisés en cosmétique. Ils peuvent notamment se recristalliser. Ils peuvent en outre se dégrader facilement à la lumière et/ou à la température, notamment à cause de phénomènes d'oxydation.
Ils perdent ainsi rapidement leur activité au cours du temps et cette instabilité va à l'encontre de l'efficacité recherchée.

L'utilisation de ces dérivés diphényl-méthane hydroxylés est également souhaitable sous forme solubilisée dans les supports cosmétiques et/ou dermatologiques car elle conduit à une meilleure biodisponibilité dans la peau que des formes cristallisées dont la taille des cristaux est mal contrôlée.

Par "biodisponibilité", on entend au sens de la présente demande la pénétration moléculaire de l'actif concerné dans les couches vivantes de la peau et en particulier de l'épiderme. On cherchera à ce que la concentration pénétrée soit la plus élevée possible, de façon à augmenter le taux d'actif arrivant jusqu'aux couches vivantes de la peau.

Par "forme solubilisée", on entend une dispersion des dérivés selon l'invention dans un liquide, à l'état moléculaire libre, en particulier sous forme non complexée. Aucune cristallisation des dérivés diphényl-méthane hydroxylés ne doit être visible à l'oeil nu ou en microscopie optique en polarisation croisée.

Par ailleurs, on cherche à éviter dans les formulations la trop grande présence de solvants huileux pour des raisons sensorielles : toucher cosmétique final de type huileux et gras.

Il existe donc le besoin de disposer de compositions, notamment cosmétiques, comprenant des dérivés diphényl-méthane hydroxylés et qui permettent d'améliorer la solubilisation, la stabilisation et/ou l'activité de ces dérivés diphényl-méthane hydroxylés dans un milieu physiologiquement acceptable, tout en présentant par ailleurs de bonnes propriétés cosmétiques, tant au niveau du toucher qu'au niveau de la tolérance et dont la conservation dans le temps ne demande pas de précautions particulières.

On entend par "milieu physiologiquement acceptable" un milieu compatible avec la peau y compris le cuir chevelu, les muqueuses, les yeux et/ou les cheveux.

C'est dans ce contexte que la Demanderesse propose des compositions comprenant (a) au moins un dérivé diphényl-méthane hydroxylé de formule (I), tel que décrit dans la demande WO2004/105736 et (b) au moins un ingrédient favorisant la solubilisation dudit dérivé diphényl-méthane hydroxylé.

Ces compositions sont avantageuses dans les domaines cosmétiques et dermatologiques, notamment pour le soin et/ou le maquillage des peaux grasses et/ou âgées, et en particulier pour le soin et/ou le maquillage des peaux foncées.

Par peaux foncées, on entend des peaux de personnes ayant un phototype élevé, c'est-à dire des peaux de personnes ayant un phototype III à VI, définis selon la classification de Fitzpatrick qui repose sur la réactivité de la peau aux effets du rayonnement solaire :

I brûle toujours, ne bronze jamais
II brûle toujours, bronze peu
III brûle modérément, bronze progressivement

IV brûle faiblement, bronze très facilement
V brûle rarement, bronze intensément
VI ne brûle jamais, fortement pigmenté

Cette association d'au moins un dérivé diphényl-méthane hydroxylé de formule (I) et d'au moins un ingrédient favorisant la solubilisation dudit dérivé sera également avantageuse pour la préparation de compositions pharmaceutiques destinées au traitement des désordres dermatologiques tels que les lésions acnéiques, en particulier des désordres hyperpigmentaires desdites lésions acnéiques.

L'invention concerne donc notamment une composition comprenant, dans un milieu physiologiquement acceptable, (a) au moins un dérivé diphényl méthane hydroxylé de formule (I) et (b) au moins un ingrédient favorisant la solubilisation dudit dérivé diphényl méthane hydroxylé de formule (I), ledit ingrédient étant choisi parmi : le N-lauroylsarcosinate d'isopropyle, le dimethyl isosorbide et le dicaprylyl carbonate.

**Dérivés diphényl-méthane hydroxylés**

Les dérivés diphényl-méthane hydroxylés utilisables dans les compositions de l'invention sont décrits dans la demande WO2004/105736.
Ces composés ont la formule (I) suivante :

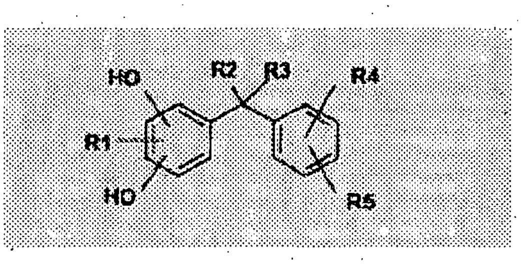

dans laquelle :

- R1 est choisi parmi un atome d'hydrogène, un groupement méthyle, une chaîne alkyle linéaire ou ramifiée, saturée ou insaturée, ayant de 2 à 4 atomes de carbone, un groupement -OH, et un halogène,
- R2 est choisi parmi un atome d'hydrogène, un groupement méthyle, une chaîne alkyle linéaire ou ramifiée, saturée ou insaturée, ayant de 2 à 5 atomes de carbone,
- R3 est choisi parmi un groupement méthyle ou une chaîne alkyle linéaire ou ramifiée, saturée ou insaturée, ayant de 2 à 5 atomes de carbone,
- R4 et R5 sont indépendemment l'un de l'autre choisi parmi un atome d'hydrogène, un groupement méthyle, une chaîne alkyle linéaire ou ramifiée, saturée ou insaturée, ayant de 2 à 5 atomes de carbone, un groupement -OH ou un halogène.

Les groupements -OH, R1, R4 et R5 peuvent être en position ortho, meta ou para par rapport à la liaison formée avec le carbone reliant entre eux les deux noyaux aromatiques.

Sont également compris dans les composés de l'invention possédant les groupes phényl substitués et pour lesquels R2 et R3 sont distincts, les formes énantiomères de configuration S, les énantiomères de configuration R ou leur mélange racémate.

L'invention concerne donc notamment une composition comprenant, dans un milieu physiologiquement acceptable, (a) au moins un dérivé diphényl méthane hydroxylé de formule (I)

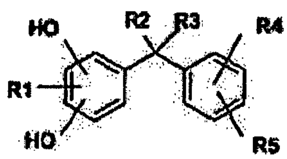

dans laquelle :

- R1 est choisi parmi un atome d'hydrogène, un groupement méthyle, une chaîne alkyle linéaire ou ramifiée, saturée

ou insaturée, ayant de 2 à 4 atomes de carbone, un groupement -OH, et un halogène,

- R2 est choisi parmi un atome d'hydrogène, un groupement méthyle, une chaîne alkyle linéaire ou ramifiée, saturée ou insaturée, ayant de 2 à 5 atomes de carbone,
- R3 est choisi parmi un groupement méthyle ou une chaîne alkyle linéaire ou ramifiée, saturée ou insaturée, ayant de 2 à 5 atomes de carbone,
- R4 et R5 sont indépendemment l'un de l'autre choisi parmi un atome d'hydrogène, un groupement méthyle, une chaîne alkyle linéaire ou ramifiée, saturée ou insaturée, ayant de 2 à 5 atomes de carbone, un groupement -OH ou un halogène

et (b) au moins un ingrédient favorisant la solubilisation dudit dérivé diphényl méthane hydroxylé de formule (I) selon la revendication 1.

Selon un mode préféré de l'invention, on utilise un composé de formule (I) dans lequel :

- R1, R2, R4 et R5 désignent un atome d'hydrogène ;
- R3 est un groupement méthyle ;
- les groupements -OH sont en position ortho et para par rapport à la liaison formée avec le carbone reliant entre eux entre les deux noyaux aromatiques.

Ce composé correspond à la formule (II) suivante

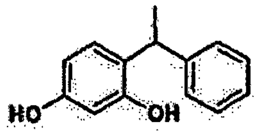

nommé 4-(1-phenylethyl)-1,3-benzenediol ou 4-(1-phenylethyl)-1,3-dihydroxybenzène ou autrement nommé phenyethyl resorcinol ou phenylethylbenzenediol ou styrylresorcinol. Ce composé a un numéro CAS 85-27-8.

Un tel composé est commercialisé sous la dénomination SYMWHITE 377® ou BIO 377 par la société SYMRISE.

Selon un mode préféré de l'invention, la composition comprend, dans un milieu physiologiquement acceptable, (a) au moins un dérivé diphényl méthane hydroxylé de formule (II)

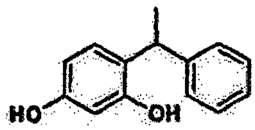

et (b) au moins un ingrédient favorisant la solubilisation dudit dérivé diphényl méthane hydroxylé de formule (II).

La quantité de composé diphényl-méthane selon l'une des formule (I) ou formule (II) présente dans la composition selon l'invention ira notamment de 0,0001 à 20% en poids par rapport au poids total de la composition, en particulier de 0,001 à 15%, de 0,001 à 10%, de 0,001 à 8%, de préférence de 0,001 à 5%, de 0,01 à 5%, de 0,01 à 4%, de 0,01 à 3%, de 0,01 à 2% et encore plus préférentiellement de 0,01 à 1% en poids par rapport au poids total de la composition.

**<u>Ingrédients favorisant la solubilisation desdits dérivés diphényl-méthane</u>**

Comme ingrédients favorisant la solubilisation desdits dérivés diphényl-méthane de formule (I) ou (II) on peut citer notamment :

**(i)** des dérivés lipophiles d'acides aminés, tels que notamment ceux décrits dans la demande de brevet EP 1 269 986, incorporée ici par référence.
En particulier, on peut utiliser comme dérivés lipophiles d'acides aminés ceux de formule

$$R'_1\ (CO)N(R'_2)CH(R'_3)(CH_2)n(CO)OR'_4$$

dans laquelle :

n est en entier égal à 0,1 ou 2,

$R'_1$ représente un radical alkyle ou alcényle en $C_5$ à $C_{21}$, linéaire ou ramifié,
$R'_2$ représente un atome d'hydrogène ou un groupe alkyle en $C_1$ à $C_3$,
$R'_3$ représente un radical choisi dans le groupe formé par un atome d'hydrogène, un groupe méthyle, un groupe éthyle, un radical alkyle linéaire ou ramifié en $C_3$ ou $C_4$,
$R'_4$ représente un radical alkyle en $C_1$ à $C_{10}$ ou alcényle en $C_2$-$C_{10}$, linéaire ou ramifié, ou un reste stérol.

Le dérivé lipophile d'acide aminé choisi pour être utilisé dans les compositions de l'invention est le N-lauroylsarcosinate d'isopropyle commercialisé notamment par AJINOMOTO sous la dénomination ELDEW SL205.

**(ii)** des alcools gras comportant de 8 à 26 atomes de carbone, tels que les alcools laurique, cétylique, myristique, stéarylique, palmitique, oléique, linoléique, le 2-octyldodécanol, et leurs mélanges tels que l'alcool cétéarylique (mélange d'alcool cétylique et d'alcool stéarylique). Comme par exemple l'octyl-2-dodécanol commercialisé sous la dénomination EUTANOL G par la société COGNIS ou sous la dénomination ISOFOL 20 N/F par la société SASOL.

**(iii)** des éthers tels que l'éther dicaprylique (nom CTFA : Dicaprylyl ether) ; ou des éthers d'isosorbide tels que le diméthyl isosorbide ; parmi ces éthers le dimethyl isosorbide commercialisé sous la dénomination ARLASOLVE DMI par la société UNIQEMA a été choisi pour être utilisé dans les compositions de l'invention.

**(iv)** des esters tels que les benzoates d'alcools gras en $C_{12}$-$C_{15}$ (Finsolv TN de FINETEX) ; ou des esters d'alcool caprylique ; parmi ces substances le dicaprylyl carbonate commercialisé sous la dénomination CETIOL CC par la société COGNIS a été choisi pour être utilisé dans les compositions de l'invention.

**(v)** des filtres UV liposolubles tels que ceux décrits dans la demande de brevet L'OREAL EP 1 221 933. On peut citer notamment

(1) les dérivés de l'acide salicylique choisis parmi le salicylate d'homomenthyle, le salicylate de 2-éthylhexyle, le salicylate de triéthanolamine, le salicylate de 4-isopropylbenzyle ;
(2) les dérivés de l'acide cinnamique tels que le 4-méthoxy cinnamate d'isopentyle, le 4-méthoxy cinnamate de 2-éthylhexyle, le diisopropyl cinnamate de méthyle, le 4-méthoxy cinnamate d'isoamyle, le 4-méthoxy cinnamate de diéthanolamine ;
(3) les dérivés de $\beta,\beta'$-diphénylacrylate liquides ;
(4) les dérivés de l'acide para-aminobenzoïque tels que le p-diméthylaminobenzoate de 2-éthylhexyle et le p-aminobenzoate de glycérol ;
(5) les dérivés de dibenzoylméthane ;
(6) les dérivés liposolubles de benzophénone ;
(7) les silicones benzotriazoles décrites notamment dans la demande de brevet EP-A-0 392 883 ;
(8) les dérivés siliciés de Benzimidazolyl-Benzazoles N-substitués ou de Benzofuranyl-Benzazoles décrits notamment dans la demande de brevet EP-1 028 120 ; et
(9) leurs mélanges.

Comme par exemple le p-méthoxycinnamate de 2-éthylhexyle, ou l'octylmethoxycinnamate vendu notamment sous le nom commercial « PARSOL MCX » par la Société GIVAUDAN (DSM Nutritional Products),
**[0027]** le 4-(tert-butyl) 4'-méthoxy dibenzoylméthane, notamment celui proposé à la vente sous la dénomination commerciale de "PARSOL 1789" par la Société GIVAUDAN,
le salicylate d'octyle vendu notamment sous la dénomination commerciale de "UVINUL O-18" par la Société BASF,
le 2-(2H-benzotriazole-2-yl)-4-méthyl-6-[2-méthyl-3-[1,3,3,3-tétraméthyl-1-[(triméthylsilyl)oxy] disiloxanyl]propynyl]phénol,
**[0028]** l'octylmethoxycinnamate vendu notamment sous le nom commercial « PARSOL MCX » par la Société GIVAUDAN (DSM Nutritional Products).
En outre, la quantité pondérale de filtre UV liposoluble peut représenter avantageusement de 0,5% à 20%, mieux, de 0,1 à 10%, du poids total de la composition.
Les solubilisants décrits précédemment peuvent être utilisés seuls ou en mélange.
Selon un mode particulier, pour augmenter l'effet de solubilisation des ingrédients (i) à (iv), on pourra en outre ajouter des filtres UV liposolubles (v) tels que ceux décrits dans la demande de brevet L'OREAL EP 1 221 933.
L'invention concerne donc une composition comprenant au moins un dérivé diphényl-méthane de formule (I) ou (II) et dans laquelle l'ingrédient favorisant la solubilisation desdits dérivés diphényl-méthane de formule (I) ou (II) est un choisi parmi le N-lauroylsarcosinate d'isopropyle, le dimethyl isosorbide, le dicaprylyl carbonate et leurs mélanges.
Selon un mode préféré, la composition selon l'invention comprend (a) au moins un dérivé diphényl méthane

hydroxylé de formule (I) ou (II) et au moins un dérivé lipophile d'acide aminé étant le N-lauroylsarcosinate d'isopropyle commercialisé notamment par AJINOMOTO sous la dénomination ELDEW SL 205.
Le N-lauroylsarcosinate d'isopropyle sera avantageux pour solubiliser de grandes quantités de dérivé diphényl méthane hydroxylé de formule (I) ou (II) pouvant aller jusqu'à 10% en poids de dérivé diphényl méthane hydroxylé de formule (I) ou (II) présent dans ladite composition

Selon un autre mode préféré, la composition selon l'invention comprend (a) au moins un dérivé diphényl méthane hydroxylé de formule (I) ou (II) et au moins un éther d'isosorbide étant le dimethyl isosorbide commercialisé sous la dénomination ARLASOLVE DMI par la société UNIQEMA.

Selon un autre mode, la composition selon l'invention comprend (a) au moins un dérivé diphényl méthane hydroxylé de formule (I) ou (II) et au moins un ester d'alcool caprylique étant le dicaprylyl carbonate commercialisé sous la dénomination CETIOL CC par la société COGNIS.
Le dicaprylyl carbonate sera avantageux dans des compositions où l'on recherche un toucher très léger.

Les ingrédients favorisant la solubilisation dudit dérivé diphényl méthane hydroxylé de formule (I) ou (II) selon l'invention seront présents dans la composition en une teneur allant généralement de 0,2% à 20% en poids par rapport au poids total de la composition, de préférence de 0,5% à 20% en poids, plus préférentiellement de 1% à 15% en poids ou encore de 2% à 10% en poids par rapport au poids total de la composition.

La composition selon l'invention peut en outre comprendre au moins un ingrédient favorisant la stabilisation et/ou l'activité dudit dérivé diphényl méthane hydroxylé.

Ingrédients favorisant la stabilisation desdits dérivés diphényl-méthane

Par ingrédient favorisant la stabilisation desdits dérivés diphényl-méthane hydroxylés de formule (I) ou (II), on entend selon l'invention notamment un ingrédient permettant (i) soit de stabiliser ledit dérivé diphényl-méthane hydroxylé, soit (ii) de stabiliser le milieu physiologiquement acceptable dans lequel est présent ledit dérivé diphényl-méthane hydroxylé.

En particulier dans le cas (ii), l'ingrédient favorisant la stabilisation desdits dérivés diphényl-méthane hydroxylés de formule (I) ou (II) peut notamment être présent sous une forme galénique particulière qui contribue à la stabilisation dudit dérivé diphényl-méthane hydroxylé dans ledit milieu physiologiquement acceptable.

Comme ingrédients de stabilisation desdits dérivés diphényl-méthane de formule (I) ou (II) utilisables dans les compositions de l'invention, on peut citer notamment :

(a) des polymères et/ou copolymères blocs ;
(b) des lipides amphiphiles de type ioniques ou non ioniques présents sous forme de vésicules en dispersion ;
(c) des polymères constitutifs de nanoparticules, en particulier de nanosphères ou de nanocapsules ;
(d) des polymères constitutifs de microparticules ;
(e) des polymères et/ou des tensioactifs formant des nanoémulsions ;
(f) des polymères sous forme de films fins ;
(g) des émulsionnants polyoléfines à partie polaire, la composition étant sous la forme d'une émulsion eau-dans-huile ;
(h) des polymères amphiphiles comprenant des motifs acide 2-acrylamido 2-méthylpropane sulfonique (AMPS).

**(a) polymères et/ou copolymères blocs**

Il est connu d'encapsuler des actifs lipophiles dans des micelles de copolymères blocs, par exemple des copolymères diblocs ou triblocs de poly(éthylène oxyde - propylène oxyde).

De façon avantageuse, on utilisera dans la composition de l'invention un copolymère amphiphile bloc comportant au moins un bloc polymérique hydrophile non ionique et au moins un bloc polymérique hydrophobe particulier.

Ces copolymères amphiphiles blocs sont notamment décrits dans la demande de brevet EP 1 555 984, incorporée ici par référence.

Le poids moléculaire du copolymère bloc est généralement compris entre 1000 et 100.000.

En particulier, le rapport en poids du ou des blocs polymériques hydrophiles ioniques ou non ioniques sur le ou les blocs polymériques hydrophobes est compris entre 1/100 et 50/1.

Le bloc polymérique hydrophobe est notamment choisi parmi :

- le styrène et ses dérivés tels que le 4-butylstyrène,
- les oxydes d'alkylène comportant plus de 4 atomes de carbone, et de préférence de 4 à 6 atomes de carbone,
- les monomères vinyliques hydrophobes de formule (A) suivante :

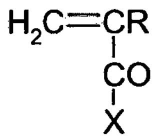

(A)

dans laquelle :

- R est choisi parmi H, $-CH_3$,
- X est choisi parmi les oxydes d'alkyle de type -OR' où R' est un radical hydrocarboné, linéaire ou ramifié, saturé ou insaturé, ayant de 1 à 22 atomes de carbone.

De préférence, le bloc polymérique hydrophobe est obtenu à partir d'un ou plusieurs monomères hydrophobes choisis parmi le méthacrylate de méthyle, le méthacrylate d'éthyle, le (méth)acrylate de n-butyle, le (méth)acrylate de tertio-butyle, l'acrylate de cyclohexyle.

En particulier, le bloc polymérique hydrophobe est choisi parmi le polystyrène, le poly(tert.-butylstyrène), le polyméthylméthacrylate, le polyéthylacrylate, le polybutylméthacrylate, les polyoxydes d'alkylène en $C_3$-$C_6$.

De préférence le bloc polymérique hydrophile non ionique est choisi parmi les polyoxydes d'éthylène.

De préférence, le copolymère bloc est choisi parmi les copolymères blocs suivants :

- polystyrène/polyoxyéthylène
- polyméthylméthacrylate/polyoxyéthyléne
- polybutylméthacrylate/polyoxyéthylène
- polyoxyéthylène/polyoxybutylène/polyoxyéthylène.

**(b) des lipides amphiphiles de type ioniques ou non ioniques ;**

Selon un autre mode de réalisation de l'invention, les dérivés diphényl-méthane de formule (I) ou (II) sont associés avec des lipides amphiphiles ioniques ou non ioniques présents sous la forme de vésicules type ionique (ex: liposomes) et/ou non ionique (ex: niosomes) en dispersion dans le milieu physiologiquement acceptable de la composition, en particulier en dispersion aqueuse.

La présence de ces vésicules contribue à la stabilisation desdits dérivés diphényl-méthane de formule (I) ou (II) dans le milieu physiologiquement acceptable de la composition.

Ces vésicules lipidiques peuvent être à coeur aqueux ou à coeur huileux.
On utilisera préférentiellement les vésicules lipidiques à coeur huileux.

Par "vésicule", on entend selon l'invention toute structure particulaire comprenant, d'une part, une membrane ou "phase lipidique" constituée par un ou plusieurs feuillets concentriques, ces feuillets comportant une ou plusieurs couches bimoléculaires à base de lipides amphiphiles ioniques ou non ioniques et, d'autre part, une phase aqueuse ou huileuse encapsulée par cette phase lipidique. Au sens de l'invention, les liposomes et les niosomes constituent notamment de telles vésicules.

Les niosomes sont des vésicules préparées à partir de lipides amphiphiles non ioniques. On peut notament se référer à la description du brevet FR 8 907 947.
Comme lipide amphiphile non ionique, on peut citer notamment les alkyl- ou polyalkylesters de polyol, éventuellement oxyéthylénés, et les éthers de polyol, éventuellement oxyéthylénés, ayant un point de fusion d'au moins 40°C.

Les liposomes sont des vésicules préparées à partir de lipides amphiphiles ioniques. Ces vésicules sont des particules formées d'une membrane constituée par un ou plusieurs feuillets concentriques, ces feuillets comportant une ou plusieurs couches bimoléculaires de lipides amphiphiles encapsulant une phase aqueuse ou huileuse. La phase aqueuse peut contenir des substances actives hydrosolubles et les couches bimoléculaires de lipides amphiphiles peuvent contenir des substances actives lipophiles. Ces vésicules ont généralement un diamètre moyen compris entre 10 et 5000 nanomètres.
Les lipides amphiphiles ioniques peuvent être des lipides amphiphiles anioniques ou des lipides amphiphiles cationiques.
Comme exemples de lipides amphiphiles anioniques, on peut citer notamment :

- des lipides anioniques neutralisés, de préférence, choisis parmi les sels alcalins du dicétylphosphate, et du dimyristylphosphate, en particulier les sels de sodium et potassium, les sels alcalins de l'acide phosphatidique, en particulier le sel de sodium, les sels alcalins du cholestérol-sulfate, en particulier le sel de sodium, les sels alcalins du cholestérol-phosphate, en particulier le sel de sodium, les sels de lipoaminoacides tels que les acylglutamates

mono- et disodiques, plus particulièrement le sel disodique de l'acide N-stéaroyl L-glutamique commercialisé sous la dénomination Acylglutamate HS21 par la société AJINOMOTO,

- des lipides amphotères, en particulier la phosphatidyléthanolamine de soja pure ;
- des dérivés alkylsulfoniques.

Comme lipides amphiphiles cationiques, on peut utiliser notamment les sels d'ammonium quaternaire, les amines grasses et leurs sels.

On peut avantageusement utiliser des compositions à 'double liposome' pour le traitement simultané des couches superficielles et profondes de la peau, comprenant une première dispersion de vésicules lipidiques aptes à pénétrer dans les couches profondes de la peau et contenant au moins un actif apte à traiter ces couches profondes et une deuxième dispersion de vésicules lipidiques aptes à pénétrer dans les couches superficielles de la peau et contenant au moins un actif apte à traiter ces couches superficielles. Un tel système est décrit dans le brevet EP 0 661 035.

Les dérivés diphényl-méthane hydroxylés de formule (I) ou (II) seront de préférence présents dans la deuxième dispersion de vésicules lipidiques aptes à pénétrer dans les couches superficielles de la peau.
Ils pourront être associés à des actifs ciblant le derme (ex : actifs anti-âge) présents dans une première dispersion de vésicules lipidiques aptes à pénétrer dans les couches profondes de la peau.

Les oléosomes sont des globules huileux pourvus d'un enrobage cristal liquide lamellaire dispersés dans une phase aqueuse, de diamètre moyen généralement inférieur à 500 nanomètres.
Comme exemple de formulation dans des oléosomes, on peut se référer notamment au brevet EP0 641 557.

**(c) des polymères constitutifs de nanoparticules**

Selon une alternative, avantageuse pour les actifs à caractère lipophile tels que les dérivés diphényl-méthane hydroxylés de formule (I) ou (II), on pourra associer lesdits actifs à des particules de petites tailles, appelées notamment nanoparticules.
Il peut s'agir de particules solides constituées de l'association dudit dérivé diphényl-méthane hydroxylé de formule (I) ou (II) et d'au moins un polymère.
Ce polymère contribue à la stabilisation dudit dérivé diphényl-méthane hydroxylé de formule (I) ou (II) dans le milieu physiologiquement acceptable de la composition.

Le terme de "nanoparticules" englobe principalement deux systèmes différents : des "nanosphères" constituées d'une matrice polymère dans laquelle ledit dérivé diphényl-méthane hydroxylé de formule (I) ou (II) est absorbé et/ou adsorbé et/ou mélangé, ainsi que des "nanocapsules" ayant une structure de type noyau-enveloppe, c'est-à-dire une structure constituée d'un coeur lipidique liquide à température ambiante contenant dérivé diphényl-méthane hydroxylé de formule (I) ou (II) sous forme solubilisée, lequel coeur est encapsulé dans une enveloppe protectrice continue insoluble dans le milieu.
On utilisera de préférence des nanocapsules.

On pourra se référer par exemple à la description de la demande de brevet EP 1 414 390.

Les nanosphères ont généralement une taille moyenne comprise entre 50 et 500 nm.

Les nanocapsules sont généralement de petite taille afin d'obtenir une biodisponibilité optimale des dérivés diphényl-méthane hydroxylés de formule (I) ou (II).
De façon préférentielle, la taille de ces nanocapsules est comprise entre 10 nm et 1000 nm et plus particulièrement entre 30 nm et 500 nm.
On pourra notamment utiliser des polymères sous forme de nanocapsules telles que décrites dans la demande de brevet EP-0274961, les nanocapsules pourvues d'un enrobage lamellaire décrites dans la demande EP-0780115, les nanocapsules dont l'enveloppe polymérique continue insoluble dans l'eau est constituée de polyesters tel que décrit dans les demandes EP-1025901, FR-2787730, et EP-1034839, ou bien encore les nanocapsules biodégradables décrites dans la demande de brevet FR-2 659 554, ou non biodégradables décrites dans la demande de brevet WO-93/05753.

Les nanocapsules en polymères biodégradables pénètrent dans la peau et se dégradent dans l'épiderme sous l'action des enzymes qui y sont présentes, tandis que les nanocapsules en polymères non biodégradables ne pénètrent que dans les couches superficielles du *stratum corneum* et sont éliminées naturellement lors du renouvellement de la peau.

Comme polymères constitutifs de nanocapsules utilisables dans les compositions de l'invention en association avec les dérivés diphényl-méthane hydroxylés de formule (I) ou (II), on peut citer notamment les poly-L- et DL-lactides et les polycaprolactones, les polyglycolides et leurs copolymères, les polymères issus de la polymérisation d'alkyl cyanoacrylate (la chaîne alkyle ayant de 2 à 6 carbone) ; les polymères anioniques hydrodispersibles synthétiques ou naturels ; les polyesters du type poly(alkylène adipate) ; les polymères dendritiques ; les copolymères de chlorure de vinyle et d'acétate de vinyle, les copolymères d'acide méthacrylique et d'ester méthylique d'acide méthacrylique, l'acéto-phtalate de polyvinyle, l'acéto-phtalate de cellulose, les copolymères de polyvinylpyrrolidone-acétate de vinyle réticulé,

les polyéthylènevinylacétates, les polyacrylonitriles, les polyacrylamides , les polyéthylèneglycols, les polyamides, les polyéthylènes, les polypropylènes et les organopolysiloxanes.

De façon préférée, on utilisera des polycaprolactones.

**(d) des polymères constitutifs de microparticules ;**

Selon un mode de réalisation particulier de l'invention, avantageux notamment pour le soin des peaux grasses, on associera les dérivés diphényl-méthane hydroxylés de formule (I) ou (II) à des polymères constitutifs de microparticules.

Ces polymères contribuent à la stabilisation dudit dérivé diphényl-méthane hydroxylé de formule (I) ou (II) dans le milieu physiologiquement acceptable de la composition.

Le terme 'microparticules' englobe notamment les 'particules poreuses' et en particulier les 'microsphères'.

Par l'expression « particules poreuses », on désigne des particules ayant une structure comportant des pores. Cette structure poreuse peut permettre au moins en partie l'incorporation d'un ou plusieurs agents actifs au sein desdites particules.

On peut se référer notamment à la demande de brevet EP 1 637 124.

La structure des particules peut être de type matriciel à l'image d'une éponge. Elle peut également être de type vésiculaire, c'est-à-dire que la particule présente une cavité interne délimitée par une paroi poreuse. La porosité associée à la taille des particules est caractérisée quantitativement par leur surface spécifique.

On utilisera notamment des particules poreuses ayant une surface spécifique mesurée selon la méthode BET supérieure ou égale à 1 $m^2/g$. La méthode BET (BRUNAUER-EMMET-TELLER) est une méthode bien connue de l'homme du métier. Elle est notamment décrite dans « The journal of the American Chemical Society », vol.60, page 309, février 1938 et correspond à la norme internationale ISO 5794/1 (annexe D). La surface spécifique mesurée selon la méthode BET correspond à la surface spécifique totale, c'est-à-dire qu'elle inclue la surface formée par les pores.
Selon un mode de réalisation particulier, les particules de l'invention présentent une surface spécifique mesurée par BET, allant notamment de 2,5 à 100 $m^2/g$.

Les particules poreuses utilisables dans les compositions de l'invention sont généralement des particules individualisées. Par l'expression « particules individualisées », on désigne des particules qui ne sont pas regroupées sous la forme d'agrégat ou d'agglomérat. Ces particules présentent en particulier une masse volumique supérieure ou égale à 0,15 $g/cm^3$ et notamment allant de 0,2 à 5 $g/cm^3$.

Ces particules ont de préférence un diamètre moyen en volume inférieur ou égal à 10 $\mu$m. En effet, de telles particules peuvent pénétrer dans le follicule sébacé par application d'une force mécanique. Cette force mécanique provient généralement d'un massage qui, outre la poussée qu'elle exerce, génère un effet de pompe au niveau du follicule. Les particules atteignent ainsi progressivement le canal folliculaire dans lequel elles sont susceptibles d'absorber le sébum et, le cas échéant, de libérer le composé actif qu'elles portent. Le matériau constitutif des particules est ensuite rejeté grâce à l'écoulement de sébum et/ou à la pousse du poil permettant ainsi d'éviter toute réaction indésirable éventuelle de l'organisme vis-à-vis de ce matériau.
En particulier, on utilisera des particules, notamment sphériques, poreuses, de dimension moyenne en nombre pouvant aller de 0,1 à 50 $\mu$m, notamment de 0,1 à 20 $\mu$m et tout particulièrement de 0,5 à 10 $\mu$m.

Comme particules poreuses préférées, on peut utiliser des particules de polyamide, en particulier de Nylon 6, Nylon 6-6, Nylon 12 ou Nylon 6-12 telles que celles commercialisées par la société ATOFINA sous le nom générique d'« Orgasol ».
Ce système d'encapsulation qui permet un ciblage folliculaire est particulièrement avantageux dans des compositions destinées au traitement des peaux grasses.

**(e) des polymères et/ou tensioactifs formant des nanoémulsions**

Selon un autre mode de réalisation de l'invention, lesdits dérivés de diphényl-méthane hydroxylés de formule (I) ou (II) sont associés dans la composition à des polymères et/ou des tensioactifs particuliers, la composition étant sous la forme d'une nanoémulsion.
Ces polymères et/ou tensioactifs contribuent à la stabilisation dudit dérivé diphényl-méthane hydroxylé de formule (I) ou (II) dans le milieu physiologiquement acceptable de la composition.

Les nanoémulsions sont généralement des émulsions huile-dans-eau dont les globules d'huile ont une granulométrie très fine, c'est-à-dire une taille moyenne en nombre, inférieure à 100 nanomètres (nm).
On peut se référer notamment à la description du brevet EP 0 728 460.

Les nanoémulsions peuvent être stabilisées par un enrobage cristal liquide lamellaire obtenu par l'association d'un tensioactif hydrophile et d'un tensioactif lipophile

Avantageusement, les polymères particuliers permettant la réalisation de nanoémulsions peuvent être choisis

parmi :

- les polymères anioniques à chaîne hydrophobe, tels que décrits dans le brevet EP 116 005; et/ou
- les polymères non ioniques hydrosolubles tels que décrits dans le brevet EP 1 172 077.

Les tensioactifs particuliers permettant la réalisation de nanoémulsions peuvent être notamment un système ternaire de tensioactifs dont un mélange de tensioactifs non ioniques et un tensioactif ionique, tels que décrits dans le brevet EP 1 353 629 incorporé ici par référence.

En particulier, le polymère anionique à chaîne hydrophobe comprend des chaînes hydrophobes choisies parmi les chaînes hydrocarbonées linéaires ou ramifiées, saturées ou insaturées, ayant de 6 à 30 atomes de carbone, les groupements divalents cycloaliphatiques et les groupements divalents aromatiques, et de préférence choisies parmi les chaînes alkyle, arylalkyle, alkylaryle, alcoylène, méthylènedicyclohexyl, isophorone et phénylène.

Notamment, le polymère anionique est choisi parmi les copolymères d'acide acrylique ou méthacrylique, les copolymères d'acide 2-acrylamido-2-méthylpropane sulfonique et leurs mélanges.

De préférence, le polymère anionique est obtenu par copolymérisation d'un monomère (a) choisi parmi les acides carboxyliques à insaturation $\alpha$ ,$\beta$-éthylénique (monomère a') et l'acide 2-acrylamido 2-méthylpropane sulfonique (monomère a"), avec un monomère (b) à insaturation éthylénique non tensioactif différent de (a) et/ou un monomère (c) à insaturation éthylénique issu de la réaction d'un monomère acrylique à insaturation $\alpha$,$\beta$-monoéthylénique ou d'un monomère isocyanate à insaturation monoéthylénique avec un composant amphiphile non ionique monohydrique ou avec une amine grasse primaire ou secondaire.

Avantageusement, le polymère anionique est un terpolymère acrylique obtenu à partir de (a) un acide carboxylique à insaturation $\alpha$,$\beta$-éthylénique, (b) un monomère à insaturation éthylénique non tensioactif différent de (a), et (c) un monomère uréthanne non-ionique qui est le produit de réaction d'un composé amphiphile non-ionique monohydrique avec un isocyanate à insaturation monoéthylénique.

Par exemple , le polymère anionique est choisi parmi le terpolymère acide acrylique/acrylate d'ethyle/acrylate d'alkyle, le copolymère acrylates/steareth-20 methacrylate, le terpolymère acide (meth)acrylique / acrylate d'éthyle / methacrylate de béhényle oxyéthyléné (25 OE), le copolymère acide acrylique/itaconate de mono-cétyle oxyéthyléné (20 OE), le copolymère acide acrylique/itaconate de mono-stéaryle oxyéthyléné (20 OE), le copolymère acrylates/acrylate modifié par des alcools en C12-C24 polyoxyéthylénés (25 OE), le terpolymère acide methacrylique/acrylate de méthyle/diméthylmétaisopropényl benzylisocyanate d'alcool béhénylique éthoxylé, et leurs mélanges.

En particulier, le polymère non ionique hydrosoluble peut être choisi parmi les homopolymères et copolymères d'oxyde d'éthylène ; les alcools polyvinyliques ; les homopolymères et copolymères de vinylpyrrolidone ; les homopolymères et copolymères de vinylcaprolactame ; les homopolymères et copolymères de polyvinylméthyléther ; les homopolymères et copolymères acryliques neutres ; les alkyl-$C_1$-$C_2$-celluloses et leurs dérivés ; les alkyl-$C_1$-$C_3$-guar ou hydroxyalkyl-$C_1$-$C_3$-guar.

Le système ternaire de tensioactifs pourra comprendre notamment :

(a) un mélange d'au moins deux tensioactifs non ioniques comprenant au moins un ester gras éthoxylé comportant 8 à 100 unités (notamment 10 à 80 unités, et mieux 40 ) d'oxyde d'éthylène et au moins un ester d'acide gras de sorbitane ; et
(b) au moins un tensioactif ionique choisi parmi les sels alcalins de cétylphosphate et les sels alcalins de palmitoyl sarcosinate.
L'ester gras éthoxylé est de préférence le stéarate de polyéthylèneglycol 40 OE et l'ester d'acide gras de sorbitane est de préférence le tristéarate de sorbitane.
Le tensioactif ionique est notamment choisi parmi le cétyl phosphate de potassium, le palmitoyl sarcosinate de sodium et leurs mélanges.

**(f) des polymères sous forme de films fins**

Selon un autre mode de réalisation de l'invention, lesdits dérivés diphényl-méthane hydroxylés de formule (I) ou (II) sont associés à au moins un polymère hydrosoluble ou hydrodispersible sous forme d'un film fin.
La composition se présente donc sous la forme d'un film fin.

Ce polymère hydrosoluble ou hydrodispersible contribue à la stabilisation dudit dérivé diphényl-méthane hydroxylé de formule (I) ou (II) dans le milieu physiologiquement acceptable de la composition.

Par "film", on entend dans la présente demande, un solide fin et préhensible. On entend par "fin", un solide ayant une épaisseur d'au maximum 1000 $\mu$m. Ce film a généralement une dimension adéquate pour pouvoir être

facilement manipulé par l'utilisateur. Il peut avoir une forme de carré, de rectangle, de disque ou toute autre forme. Chaque film a généralement une épaisseur de 10 μm à 1000 μm, de préférence de 20 à 500 μm et mieux de 50 à 300 μm. Il peut avoir une surface de 0,25 à 25 $cm^2$ et de préférence de 2 à 10 $cm^2$.
Ces films fins contiennent généralement moins de 10 % en poids d'eau, de préférence moins de 5 % en poids par rapport au poids total du film, et de préférence encore, ne contenant pas d'eau.

De tels films sont décrits dans la demande de brevet EP1588694, incorporée ici par référence.

Le film fin comprend un polymère hydrosoluble ou hydrodispersible qui peut être choisi parmi : (1) les polymères de type protéique, tels que les protéines de blé ou de soja ; la kératine, par exemple les hydrolysats de kératine et les kératines sulfoniques ; la caséine ; l'albumine ; le collagène ; la gluteline ; le glucagon ; le gluten ; la zéine ; les gélatines et leurs dérivés ; (2) les polymères dérivant de la chitine ou du chitosane, tels que les polymères anioniques, cationiques, amphotères ou non ioniques de chitine ou de chitosane ; (3) les polymères polysaccharidiques tels que notamment (i) les polymères cellulosiques, comme l'hydroxyéthylcellulose, l'hydroxypropylcellulose, l'hydroxypropylméthylcellulose, la méthylcellulose, l'éthylhydroxyéthylcellulose, la carboxyméthylcellulose, et les dérivés quaternisés de la cellulose ; et (ii) les amidons et leurs dérivés ; (4) les polymères ou copolymères acryliques tels que les polyacrylates, les polyméthacrylates et leurs copolymères ; (5) les polymères vinyliques tels que les polyvinylpyrrolidones, les copolymères du méthylvinyléther et de l'anhydride maléique, le copolymère de l'acétate de vinyle et de l'acide crotonique, les copolymères de la vinylpyrrolidone et de l'acétate de vinyle, les copolymères de la vinylpyrrolidone et du caprolactame, les alcools polyvinyliques ; (6) les polymères d'origine naturelle, éventuellement modifiés, tels que la gomme arabique, la gomme de guar, les dérivés du xanthane, la gomme de karaya ; les alginates, les carraghénanes, les ulvanes et autres colloïdes algaux ; les glycoaminoglycanes, l'acide hyaluronique et ses dérivés ; la gomme laque, la gomme sandaraque, les dammars, l'élémis, les copals ; l'acide désoxyribonucléique ; les mucopolysaccharides, tels que l'acide hyaluronique, le sulfate de chondroïtine ; et les mélanges de ces polymères.

On peut encore citer comme polymères hydrosolubles, les caprolactames, la pullulane, la pectine, la mannane et les galactomannanes, les glucomannanes et leurs dérivés.

De préférence, le polymère hydrosoluble peut être un polymère cellulosique, en particulier l'hydroxypropyl cellulose ou l'hydroxypropyl méthyl cellulose, ou bien encore un alginate, notamment l'alginate de sodium.

La composition selon l'invention peut être sous la forme d'une émulsion et pourra également des émulsionnants spécifiques capables de stabiliser les dérivés diphényl-méthane hydroxylés de formule (I) ou (II) et conférer avantageusement des propriétés sensorielles remarquables, tel qu'un toucher léger et frais.

Comme exemples d'émulsionnants particuliers, on peut citer notamment :

- les émulsionnants polyoléfines à partie polaire, dans une émulsion eau-dans-huile ;
- les polymères amphiphiles comprenant des motifs acide 2-acrylamido 2-méthylpropane sulfonique (AMPS), dans une émulsion huile-dans-eau.

**<u>g) émulsionnants polyoléfines à partie polaire</u>**

L'invention concerne donc également une composition sous la forme d'une émulsion eau-dans-huile comprenant au moins un dérivé diphényl-méthane hydroxylé de formule (I) ou (II) et au moins une polyoléfine à partie polaire.

Les polyoléfines à partie polaire contribuent à la stabilisation dudit dérivé diphényl-méthane hydroxylé de formule (I) ou (II) dans le milieu physiologiquement acceptable de la composition.

Les polyoléfines à partie(s) polaire(s) utilisées dans la composition de l'invention sont généralement constituées d'une partie apolaire polyoléfinique et d'au moins une partie polaire. La partie apolaire polyoléfinique comprend au moins 40 atomes de carbone et de préférence de 60 à 700 atomes de carbone. Cette partie apolaire peut être choisie parmi les polyoléfines telles que les oligomères, les polymères et/ou les copolymères d'éthylène, d'éthylène, de propylène, de 1-butène, d'isobutène, de 1-pentène, de 2-méthyl-1-butène, de 3-méthyl-1-butène, de 1-héxène, de 1-heptène, de 1-octène, de 1-décène, de 1-undécène, de 1-dodécène, de 1-tridécène, de 1-tetradécène, de 1-pentadécène, de 1-hexadécène, de 1-heptadécène et de 1-octadécène. Ces polyoléfines sont hydrogénées ou non.

La partie polaire des polyoléfines à partie polaire peut être anionique, cationique, non ionique, zwitterionique ou amphotère. Elle est par exemple constituée de polyalkylène-glycols (notamment polyoxyéthylène ) ou de polyalkylène-imines, ou encore d'acides ou de diacides carboxyliques, de leurs anhydrides ou de leurs dérivés tels que leurs esters, leurs amides et leurs sels, et leurs mélanges. Les polyoléfines à partie polaire acide carboxylique peuvent être par exemple issues de la réaction entre une polyoléfine et au moins un acide ou anhydride carboxylique éventuellement totalement ou partiellement salifié, choisi dans le groupe comprenant l'acide ou l'anhydride succinique, l'acide maléique, l'anhydride maléique, l'acide fumarique, l'acide itaconique, l'acide citraconique (ou acide méthyl maléique), l'acide mésaconique (ou acide méthyl fumarique), l'acide aconitique, leurs dérivés esters ou amides, et leurs mélanges.

De préférence, la partie polaire de la polyoléfine est choisie dans le groupe comprenant le polyoxyéthylène, l'acide ou l'anhydride succinique, les esters ou amides de l'acide ou de l'anhydride succinique, les sels de métal alcalin

ou alcalino-terreux ou sels organiques de l'acide ou de l'anhydride succinique, ou les sels partiels des monoesters ou monoamides de l'acide ou de l'anhydride succinique.

Les polyoléfines à partie polaire préférées dans les composition de l'invention sont des polyoléfines à terminaison succinique éventuellement modifiée, telles que décrites dans le brevet EP 1 172 089, incorporée ici par référence.

Comme polyoléfines à terminaison succinique, on peut citer notamment les polyisobutylènes à terminaison succinique estérifiée éventuellement modifiée, notamment estérifiée par la diéthanolamine, et leurs sels, notamment les sels de diéthanolamine, tels que les produits commercialisés sous les dénominations Lubrizol® 2724, Lubrizol® 2722 et Lubrizol® 5603 par la société Lubrizol.

Une autre polyéoléfine à partie polaire particulièrement préférée est un ester de polyisobutényl succinate de diéthanolaminoéthyle et de triéthanolamnine. Ce produit est vendu par exemple sous la dénomination Chemccinate® 2000 par la société Chemron.

Comme polyoléfine à partie polaire, on peut également utiliser un ester de polyisobutényl succinate de glycéryle, notamment celui vendu sous la dénomination Chemccinate® 1000 AF par la société Chemron.

**<u>h) polymères amphiphiles comprenant des motifs AMPS</u>**

Selon un autre mode de réalisation de l'invention, la composition selon l'invention est sous la forme d'une émulsion huile-dans-eau et comprend au moins un dérivé diphényl-méthane hydroxylé de formule (I) ou (II) et au moins un polymère amphiphile comprenant des motifs acide 2-acrylamido 2-méthylpropane sulfonique (AMPS).

Ce polymère amphiphile comprenant des motifs acide 2-acrylamido 2-méthylpropane sulfonique (AMPS) contribue à la stabilisation dudit dérivé diphényl-méthane hydroxylé de formule (I) ou (II) dans le milieu physiologiquement acceptable de la composition.

Comme polymère amphiphile comprenant des motifs acide 2-acrylamido 2-méthylpropane sulfonique (AMPS) utilisable dans les compositions de l'invention, on peut citer ceux décrits dans la demande de brevet EP 1 466 587.

Comme polymères amphiphiles préférés, on peut citer les copolymères d'AMPS et de méthacrylate d'alcool en $C_{12}$-$C_{14}$ oxyéthyléné, comportant notamment de 7 à 23 groupes oxyéthylénés.

Les ingrédients favorisant la stabilisation dudit dérivé diphényl méthane hydroxylé de formule (I) ou (II) selon l'invention seront présents dans la composition en une teneur allant généralement de 0,01% à 30% en poids par rapport au poids total de la composition, de préférence de 0,05% à 15% en poids, plus préférentiellement de 0,1% à 10% en poids et plus particulièrement de 0,1 à 5% en poids par rapport au poids total de la composition

Les compositions selon l'invention telles que définies précédemment et selon le mode de réalisation choisi (choix de l'ingrédient favorisant la solubilisation des dérivés de formule (I) ou (II)), peuvent se présenter sous toutes les formes galéniques classiquement utilisées pour une application topique et notamment sous forme de solutions aqueuses, hydroalcooliques, d'émulsions huile-dans-eau (H/E) ou eau-dans-huile (E/H) ou multiple (triple : E/H/E ou H/E/H), de gels aqueux, ou de dispersions d'une phase grasse dans une phase aqueuse à l'aide de nanoparticules polymériques telles que les nanosphères et les nanocapsules, ou de vésicules lipidiques de type ionique et/ou non ionique (liposomes, niosomes, oléosomes), de nanoémulsions, ou de films fins. Ces compositions sont préparées selon les méthodes usuelles.

En outre, les compositions utilisées selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick.

Quand la composition utilisée selon l'invention comporte une phase huileuse, celle-ci contient de préférence au moins une huile. Elle peut contenir en outre d'autres corps gras.

Comme huiles utilisables dans la composition de l'invention, on peut citer par exemple :

- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules $R^1COOR^2$ et $R^1OR^2$ dans laquelle $R^1$ représente le reste d'un acide gras comportant de 8 à 29 atomes de carbone, et $R^2$ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate,

l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;

- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényldiméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Selon un mode particulier de réalisation de l'invention, la composition selon l'invention est une émulsion eau-dans-huile (E/H) ou huile-dans-eau (H/E). La proportion de la phase huileuse de l'émulsion peut aller de 5 à 80 % en poids, et de préférence de 5 à 50 % en poids par rapport au poids total de la composition.

Les émulsions contiennent généralement au moins un émulsionnant choisi parmi les émulsionnants amphotères, anioniques, cationiques ou non ioniques, utilisés seuls ou en mélange, et éventuellement un co-émulsionnant. Les émulsionnants sont choisis de manière appropriée suivant l'émulsion à obtenir (E/H ou H/E). L'émulsionnant et le co-émulsionnant sont généralement présents dans la composition, en une proportion allant de 0,3 à 30 % en poids, et de préférence de 0,5 à 20 % en poids par rapport au poids total de la composition.

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination Abil EM 90$^{R}$ par la société Goldschmidt. On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

De façon connue, la composition cosmétique ou dermatologique de l'invention peut contenir également des adjuvants habituels dans le domaine cosmétique ou dermatologique, tels que les gélifiants hydrophiles ou lipophiles, les conservateurs, les solvants, les parfums, les charges, les filtres UV, les bactéricides, les absorbeurs d'odeur, les

matières colorantes, les extraits végétaux, les sels, les antioxydants, les agents basiques, les acides, les tensioactifs non ioniques, anioniques, cationiques.
Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les vésicules lipidiques.

Comme charges qui peuvent être utilisées dans la composition de l'invention, on peut citer par exemple, outre les pigments, la poudre de silice ; le talc ; les particules de polyamide et notamment celles vendues sous la dénomination ORGASOL par la société Atochem ; les poudres de polyéthylène ; les micro-sphères à base de copolymères acryliques, telles que celles en copolymère diméthacrylate d'éthylène glycol/methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; et leurs mélanges. Ces charges peuvent être présentes dans des quantités allant de 0 à 20 % en poids et de préférence de 1 à 10 % en poids par rapport au poids total de la composition.

Comme gélifiants hydrophiles ou lipophiles, on peut citer notamment les carbopol, les luvigel, l'Hostacerin AMPS, le Simulgel, les Sepigel, les gommes de xanthane, de guar, de cellulose, les alginates et leurs mélanges. On peut citer aussi les hectorites.

Comme agents antioxydants, on peut citer notamment les polyphénols, l'acide tannique, l'épоigallocathéchines et les extraits naturels en contenant, les anthocyanes, les extraits de romarin, les extraits de feuilles d'olivier, le thé vert, le resvératrol et ses dérivés, le Pycnogénol, l'ergothinéine, la N acétylcystéine, la biotine, les chélatants, l'idébénone, des extraits végétaux come le Pronalen Bioprotect TM de la société Provital, les antiradicalaires comme la vitamine E, le co enzyme Q10, les bioflavonoides, les SOD, le phytantriol, les lignanes, la mélatonine, les pidolates, le gluthation.

Selon un mode préféré de réalisation de l'invention, la composition utilisée selon l'invention contient au moins un filtre UV (ou filtre solaire) qui peut être un filtre chimique
ou un filtre physique ou un mélange de tels filtres.

A titre d'illustration et de façon non limitative, on peut citer les familles suivantes (les noms correspondent à la nomenclature CTFA des filtres) :

les anthranilates, en particulier l'anthranilate de menthyle ; les benzophénones, en particulier la benzophénone-1, la benzophénone-3, la benzophénone-5, la benzophénone-6, la benzophénone-8, la benzophénone-9, la benzophénone-12, et préférentiellement la Benzophénone-2 (Oxybenzone), ou la Benzophénone-4 (Uvinul MS40 disponible chez B.A.S.F.) ; les benzylidènes-camphres, en particulier le 3-benzylidène-camphre, l'acide benzylidènecamphosulfonique, le benzalkoniumméthosulfate de Camphre, le polyacrylamidométhylbenzylidène camphre, l'acide téréphthalylidène di-camphre sulfonique , et préférentiellement le 4-méthylbenzylidène camphre (Eusolex 6300 disponible chez Merck) ; les benzimidazoles, en particulier le benzimidazilate (Neo Heliopan AP disponible chez Haarmann et Reimer), ou l'acide phénylbenzimidazole sulfonique (Eusolex 232 disponible chez Merck) ; les benzotriazoles, en particulier le drométrizole trisiloxane, ou le méthylène bis-benzotriazolyltétraméthylbutylphénol (Tinosorb M disponible chez Ciba) ; les cinnamates, en particulier le cinoxate, le DEA méthoxycinnamate, le méthylcinnamate de diisopropyle, le glycéryl éthylhexanoate de diméthoxycinnamate, le méthoxycinnamate d'isopropyle, le cinnamate d'isoamyle, et préférentiellement l'éthocrylène (Uvinul N35 disponible chez B.A.S.F.), l'octylméthoxycinnamate (Parsol MCX disponible chez Hoffmann La Roche), ou l'octocrylène (Uvinul 539 disponible chez B.A.S.F.) ; les dibenzoylméthanes, en particulier le butyl méthoxydibenzoylméthane (Parsol 1789) ; les imidazolines, en particulier l'éthylhexyl diméthoxybenzylidène dioxoimidazoline ; les PABA, en particulier l'éthyl Dihydroxypropyl PABA, l'éthylhexyldiméthyl PABA, le glycéryl PABA, le PABA, le PEG-25 PABA, et préférentiellement la diéthylhexylbutamido-triazone (Uvasorb HEB disponible chez 3V Sigma), l'éthylhexyltriazone (Uvinul T150 disponible chez B.A.S.F.), ou l'éthyl PABA (benzocaïne) ; les salicylates, en particulier le salicyclate de dipropylèneglycol, le salicylate d'éthylhexyle, l'homosalate, ou le TEA salicylate ; les triazines, en particulier l'anisotriazine (Tinosorb S disponible chez Ciba) ; le drometrizole trisiloxane, l'oxyde de zinc, le dioxyde de titane, l'oxyde de zinc, de fer, de zirconium, de cerium enrobés ou non.
La quantité de filtres dépend de l'utilisation finale souhaitée. Elle peut aller par exemple de 1 à 20% en poids et mieux de 2 à 10% en poids par rapport au poids total de la composition.
Les compositions selon l'invention pouront être appliquées directement sur la peau ou, de façon alternative, sur des supports cosmétiques ou dermatologiques de type occlusif
ou non occlusif, destinés à être appliqués de façon localisée sur la peau.
Le support peut être un support 'occlusif'. A titre d'exemple, le support est constitué d'un matériau thermoplastique, choisi parmi les polyéthylènes haute et basse densité, les polypropylènes, les polychlorures de vinyle, les copolymères d'éthylène et d'acétate de vinyle, les polyesters et les polyuréthanes, ou d'un complexe de tels matériaux.

Ces matériaux peuvent également être présents sous forme stratifiée avec au moins une feuille de métal telle qu'une feuille d'aluminium.

La couche support peut être de toute épaisseur appropriée qui procurera les fonctions de support et de protection souhaitées. De préférence, l'épaisseur de la couche support est comprise entre environ 20 μm et environ 1,5 mm. Avantageusement, la couche support est suffisamment flexible de manière à pouvoir épouser parfaitement le profil de la peau, et à ne pas provoquer chez l'utilisateur, une sensation d'inconfort.

De préférence toutefois, le support est 'non occlusif'. Dans cette dernière hypothèse, on utilise avantageusement un support constitué d'un papier, d'un matériau thermoplastique poreux ou perforé, d'un tissé, d'un non tissé, d'un non tissé perforé.

Lesdites compositions selon l'invention peuvent être associées à des compositions administrées par voie orale, contenant des actifs cosmétiques additionnels à effet bénéfique sur l'aspect de la peau, tels que par exemple des actifs additionnels destinés à lutter contre les signes du vieillissement cutané ou des actifs additionnens destinés à lutter contre la peau grasse.

L'ingrédient additionnel utilisé dans la composition de l'invention peut également être un ingrédient et/ou un actif cosmétique ou pharmaceutique, en particulier des ingrédients et/ou des actifs bénéfiques pour l'aspect et/ou la texture de la peau.

Selon un mode particulier de l'invention, la composition selon l'invention comprend au moins un ingrédient de solubilisation dudit dérivé diphényl-méthane hydroxylé selon la revendication 1 et en outre au moins un autre ingrédient et/ou actif additionnel choisi parmi un ingrédient et/ou actif cosmétique ou pharmaceutique.

En particulier, la composition selon l'invention comprend au moins un ingrédient de solubilisation dudit dérivé diphényl-méthane hydroxylé selon la revendication 1 et au moins un autre ingrédient et/ou actif additionnel choisi parmi un ingrédient et/ou actif cosmétique ou pharmaceutique.

Selon un mode préféré, la composition selon l'invention comprend (a) au moins un dérivé diphényl méthane hydroxylé de formule (I) ou (II), (b) au moins un dérivé lipophile d'acide aminé étant le N-lauroylsarcosinate d'isopropyle commercialisé notamment par AJINOMOTO sous la dénomination ELDEW SL 205 et au moins un autre ingrédient et/ou actif additionnel choisi parmi un ingrédient et/ou actif cosmétique ou pharmaceutique.

Selon un autre mode préféré, la composition selon l'invention comprend (a) au moins un dérivé diphényl méthane hydroxylé de formule (I) ou (II), (b) au moins un éther d'isosorbide, étant le dimethyl isosorbide commercialisé sous la dénomination ARLASOLVE DMI par la société UNIQEMA et au moins un autre ingrédient et/ou actif additionnel choisi parmi un ingrédient et/ou actif cosmétique ou pharmaceutique.

Selon un autre mode, la composition selon l'invention comprend (a) au moins un dérivé diphényl méthane hydroxylé de formule (I) ou (II), (b) au moins un ester d'alcool caprylique étant le dicaprylyl carbonate commercialisé sous la dénomination CETIOL CC par la société COGNIS et au moins un autre ingrédient et/ou actif additionnel choisi parmi un ingrédient et/ou actif cosmétique ou pharmaceutique.

**<u>Actifs additionnels cosmétiques et pharmaceutiques</u>**

Comme exemples d'actifs additionnels cosmétiques ou pharmaceutiques utilisables dans les compositions de l'invention, on peut citer les actifs décrits dans les demandes WO2004/105736 et DE10324567, incorporées ici par référence.

Les actifs additionnels pourront notamment être choisis parmi les agents dépigmentants, les agents antimicrobiens, les agents antitranspirants, les chélateurs de métaux, les protéines hydrolysées, les antioxydants, les vitamines, les agents anti-inflammatoires, les agents anti-irritants ou apaisants, les agents hydratants, les extraits végétaux, les agents améliorant la fonction barrière, les agents matifiants, les charges abrasives ou agents exfoliants, les agents desquamants, les agents sébo-régulateurs, les agents cicatrisants, les agents astringents, les charges, les azurants optiques, les agents de fluorescence, les agents stimulant la synthèse de macromolécules dermiques et/ou épidermiques et/ou empêchant leur dégradation, les agents stimulant la prolifération des fibroblastes ou des kératinocytes et/ou la différenciation des kératinocytes, les agents favorisnt la fonction barrière cutanée, les agents favorisant la maturation de l'enveloppe cornée, les inhibiteurs de NO-synthases, les antagonistes des récepteurs périphériques des benzodiazépines, les agents augmentant l'activité de la glande sébacée, les agents anti-glycation, les agents dermorelaxants ou dermodécontractants, les agents favorisant la microcirculation cutanée, les agents stimulant le métabolisme énergétique des cellules, les agents tenseurs et leurs mélanges.

Comme agents humectants ou hydratants, on peut citer notamment le glycérol et ses dérivés, l'urée et ses dérivés notamment l'Hydrovance® commercialisée par National Starch, les acides lactiques, l'acide hyaluronique, les AHA, les BHA, le pidolate de sodium, le xylitol, la sérine, le lactate de sodium, l'ectoine et ses dérivés, le chitosane et ses dérivés, le collagène, le plancton, un extrait d'imperata cylindra commercialisé sous la dénomination Moist 24® par la société Sederma.

Comme agents améliorant la fonction barrière, on peut citer notamment les céramides et dérivés, les composés à base de sphingoïdes, les glycosphingolipides, les phospholipides, le cholestérol et ses dérivés, les phytostérols, les acides gras essentiels, le diacylglycérol, la 4-chromanone et dérivés de chromon, la vaseline, la lanoline, les beures de karité, le cocoa butter, et la lanoline.

Comme agents dépigmentants, on pourra citer notamment la vitamine C et ses dérivés et notamment la vit CG, CP et 3-O ethyl vitamine C, l'alpha et la béta arbutine, le lucinol et ses dérivés, l'acide kojique, le résorcinol et ses dérivés, l'acide tranexamique et ses dérivés, l'acide gentisique, l'homogentisate, le méthyl gentisate ou l'homogentisate , l'acide dioique, le D calcium panthéteine sulfonate, l'acide lipoique, l'acide ellagique, la vitamine B3, l'acide tranexamique, l'acide lipoique, l'acide linoléique et ses dérivés, les céramides et leurs homologues, les dérivés de plantes comme la camomille, la busserole, la famille des aloe (vera, ferox, bardensis), de murier, de scutellaire, sans que cette liste soit exhaustive.

Selon un mode particulier de l'invention on pourra utiliser des ingrédients et/ou actifs additionnels pour le soin des peaux grasses et/ou pour lutter contre les signes du vieillissement cutané .

L'homme du métier choisira le ou lesdits actifs en fonction de l'effet recherché sur les matières kératiniques.

Pour le soin et/ou le maquillage des peaux âgées, il choisira de préférence au moins un actif choisi parmi les agents hydratants, les agents desquamants, les agents améliorant la fonction barrière, les agents dépigmentants, les agents antioxydants, les agents dermodécontractants, les agents anti-glycation, les agents stimulant la synthèse de macromolécules dermiques et/ou épidermiques et/ou empêchant leur dégradation, les agents stimulant la prolifération des fibroblastes ou des kératinocytes et/ou la différenciation des kératinocytes, les agents favorisant la maturation de l'enveloppe cornée, les inhibiteurs de NO-synthases, les antagonistes des récepteurs périphériques des benzodiazépines (PBR), les agents augmentant l'activité de la glande sébacée, les agents stimulant le métabolisme énergétique des cellules, les agents liporestructurants et les agents favorisant la microcirculation cutanée pour le contour des yeux, et leurs mélanges.

La composition pourra comprendre en outre au moins un ingrédient tel que des charges à effet flouteur ou des agents favorisant la coloration naturelle de la peau, destiné à apporter un effet anti-âge immédiat visuel.

Selon un mode préféré, une composition anti-âge selon l'invention comprendra au moins un dérivé diphénylméthane hydroxylé de formule (I) ou (II), au moins agent hydratant, au moins un agent desquamant, au moins un actif complémentaire anti-âge, et éventuellement au moins un ingrédient destiné à apporter un effet visuel immédiat.

Pour le soin et/ou le maquillage des peaux grasses, l'homme du métier choisira de préférence au moins un actif choisi parmi les agents desquamants, agents sébo-régulateurs ou anti-séborrhéiques, les agents astringents. La composition pourra comprendre en outre au moins un ingrédient tel que des charges à effet flouteur ou des agents matifiants, destiné à apporter un effet immédiat visuel.

Une composition selon la revendication 1 destinée aux peaux grasses comprendra en plus au moins agent sébo-régulateur, au moins un agent desquamant, au moins un agent antimicrobien, et éventuellement au moins un ingrédient destiné à apporter un effet visuel immédiat.

Pour le soin et/ou le maquillage de peaux à tendance acnéique, il choisira de préférence au moins un actif choisi parmi les agents anti-microbiens, les agents cicatrisants, les agents anti-inflammatoires.

La composition pourra comprendre en outre au moins un ingrédient additionnel destiné à apporter un effet immédiat visuel ; on peut citer notamment les agents matifiants, les charges à effet flouteur, les agents fluorescents, les agents favorisant al coloration naturellement rosée de la peau et les charges abrasives ou exfoliantes..

Selon un premier mode, la composition selon la revendication 1 comprend au moins un agent hydratant.

Selon un autre mode, la composition selon la revendication 1 comprend au moins un agent desquamant.

Selon un autre mode, la composition selon la revendication 1 comprend au moins un agent améliorant la fonction barrière.

Selon un autre mode, la composition selon la revendication 1 comprend au moins un agent dépigmentant.

Selon un autre mode, la composition selon la revendication 1 comprend au moins un agent antioxydant.

Selon un autre mode, la composition selon la revendication 1 comprend au moins un dermodécontractant.

Selon un autre mode, la composition selon la revendication 1 comprend au moins un agent anti-glycation.

Selon un autre mode, la composition selon la revendication 1 comprend au moins un agent stimulant la synthèse de macromolécules dermiques et/ou épidermiques et/ou empêchant leur dégradation.

Selon un autre mode, la composition selon la revendication 1 comprend au moins un agent stimulant la prolifération des fibroblastes ou des kératinocytes et/ou la différenciation des kératinocytes.

Selon un autre mode, la composition selon la revendication 1 comprend au moins un agent favorisant la maturation de l'enveloppe cornée.

Selon un autre mode, la composition selon la revendication 1 comprend au moins un agent stimulant le métabolisme énergétique des cellules.

Selon un autre mode, la composition selon la revendication 1 comprend au moins un agent tenseur.

Selon un autre mode, la composition selon la revendication 1 comprend au moins un agent apaisant et/ou anti-irritant.

Selon un autre mode, la composition selon la revendication 1 comprend au moins un agent sébo-régulateur ou anti-séborrhéique.

Selon un autre mode, la composition selon la revendication 1 comprend au moins un agent astringent.

Selon un autre mode, la composition selon la revendication 1 comprend au moins un agent cicatrisant.

Selon un autre mode, la composition selon la revendication 1 comprend au moins un agent anti-inflammatoire.

Selon un autre mode, la composition selon la revendication 1 comprend au moins un agent antimicrobien.

Selon un autre mode, la composition selon la revendication 1 comprend au moins un agent matifiant.

Selon un autre mode, la composition selon la revendication 1 comprend au moins une charge à effet flouteur.

Selon un autre mode, la composition selon la revendication 1 comprend au moins un agent fluorescent.

Selon un autre mode, la composition selon la revendication 1 comprend au moins un agent favorisant la coloration naturellement rosée de la peau.

Selon un autre mode, la composition selon la revendication 1 comprend au moins des charges abrasives ou agents exfoliants.

De tels exemples de composés sont décrits ci-après.

**a) <u>actifs et/ou ingrédients additionnels pour le soin de la peau grasse</u>**

Une composition selon l'invention destinée au soin de la peau grasse, comprendra avantageusement un ingrédient et/ou un actif additionnel choisi parmi des agents matifiants, les charges abrasives ou agents exfoliants, les agents desquamants, les agents antimicrobiens, les agents apaisants, les agents anti-inflammatoires, les agents sébo-régulateurs, les agents antioxydants, les agents cicatrisants, les agents astringents, et leurs mélanges.
Selon un mode particulier de l'invention, la composition selon l'invention comprend au moins un ingrédient de solubilisation et/ou de stabilisation dudit dérivé diphényl-méthane hydroxylé et en outre au moins un autre ingrédient et/ou actif additionnel pour le soin de la peau grasse.
En particulier, la composition selon l'invention comprend au moins un dérivé diphényl-méthane hydroxylé de formule (I) ou (II), au moins un ingrédient de solubilisation dudit dérivé diphényl-méthane hydroxylé et en outre au moins un autre ingrédient et/ou actif additionnel pour le soin de la peau grasse.

Les ingrédients de solubilisation préférés sont décrits précédemment. En particulier, il s'agira :

- d'un dérivé lipophile d'acide aminé, en particulier le N-lauroylsarcosinate d'isopropyle commercialisé notamment par AJINOMOTO sous la dénomination ELDEW SL 205 ;
- d'un éther d'isosorbide, en particulier le dimethyl isosorbide commercialisé sous la dénomination ARLASOLVE DMI par la société UNIQEMA;
- d'un ester d'alcool caprylique, en particulier, le dicaprylyl carbonate commercialisé sous la dénomination CETIOL CC par la société COGNIS ;
- d'un dérivé de l'acide cinnamique, en le p-méthoxycinnamate de 2-éthylhexyle, ou l'octylmethoxycinnamate vendu notamment sous le nom commercial « PARSOL MCX » par la Société GIVAUDAN (DSM Nutritional Products) ;
- de l'octyl-2-dodécanol commercialisé sous la dénomination EUTANOL G par la société COGNIS ou sous la dénomination ISOFOL 20 N/F par la société SASOL ;

ou leurs mélanges.

Des exemples de tels ingrédients et/ou actifs additionnels sont décrits ci-après.

<u>Agents matifiants</u>

Par 'agent matifiant', on entend des agents destinés à rendre la peau visiblement plus mate, moins brillante. L'effet matifiant de l'agent et/ou de la composition le contenant peut notamment être évalué à l'aide d'un gonioréflectomètre, en mesurant le rapport R entre la réflexion spéculaire et la réflexion diffuse. Une valeur de R inférieure ou égale à 2 traduit généralement un effet matifiant.

L'agent matifiant pourra notamment être choisi parmi un amidon de riz ou un amidon de maïs, la kaolinite, les silices, le talc, un extrait de graines de potiron, des microbilles de cellulose, des fibres végétales, des fibres synthétiques, en particulier de polyamides, des microsphères de copolymères acryliques expansées, des poudres de polyamides, les poudres de silice, les poudres de polytétrafluoroéthylène, les poudres de résine de silicone, les poudres de copolymères acryliques, les poudres de cire, les poudres de polyéthylène, les poudres d'organopolysiloxane réticulé élastomère enrobées de résine de silicone, les poudres composites de talc/dioxyde de titane/alumine/silice, les poudres de silicates mixtes amorphes, les poudres de polymères acryliques, les particules de silicate et notamment de silicate mixte, et leurs mélanges.

Comme exemples d'agents matifiants, on peut citer notamment :

- l'amidon de riz ou de maïs, en particulier un aluminium starch octenyl succinate commercialisé sous la dénomination Dry Flo® par la société National Starch,
- la kaolinite ;
- les silices ;
- le talc ;
- un extrait de graines de potiron tel que commercialisé sous la dénomination Curbilene® par la société Indena ;
- des microbilles de cellulose telles que décrites dans la demande de brevet L'OREAL EP 1 562 562 ;
- des fibres, telles que des fibres de soie, de coton, de laine, de lin, de cellulose extraites notamment du bois, des légumes ou des algues, de polyamide (Nylon®), de cellulose modifiée, de poly-p-phénylène téréphtamide, en acrylique, de polyoléfine, de verre, de silice, d'aramide, de carbone, de Téflon®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères, les fibres synthétiques résorbables, et leurs mélanges décrites dans la demande de brevet L'OREAL EP 1 151 742 ;
- des microsphères de copolymères acryliques expansées telles que celles commercialisées par la société EXPANCEL sous les dénominations EXPANCEL 551®,
- des charges à effet optique telles que décrites dans la demande de brevet FR 2 869 796, en particulier :

  - les poudres de polyamides (Nylon®), comme par exemple les particules de Nylon 12 du type Orgasol d'Atofina de taille moyenne 10 microns et d'indice de réfraction 1,54,
  - les poudres de silice, comme par exemple les Silica beads SB150 de Miyoshi de taille moyenne 5 microns et d'indice de réfraction 1,45,
  - les poudres de polytétrafluoroéthylène, comme les PTFE ceridust 9205F de Clariant de taille moyenne 8 microns et d'indice de réfraction 1,36,
  - les poudres de résine de silicone comme les Silicon resin Tospearl 145A de GE Silicone de taille moyenne 4,5 microns et d'indice de réfraction 1,41,
  - les poudres de copolymères acryliques, notamment de poly(méth)acrylate de méthyle, comme les particules PMMA Jurymer MBI de Nihon Junyoki de taille moyenne 8 microns et d'indice de réfraction 1,49, ou les particules Micropearl M100® et F 80 ED® de la société Matsumoto Yushi-Seiyaku ;
  - les poudres de cire comme les particules Paraffin wax microease 114S de micropowders de taille moyenne 7 microns et d'indice de réfraction 1,54,
  - les poudres de polyéthylène, notamment comprenant au moins un copolymère éthylène/acide acrylique, et en particulier constituées de copolymères éthylène/acide acrylique comme les particules Flobeads EA 209 de Sumitomo (de taille moyenne 10 microns et d'indice de réfraction 1,48),
  - les poudres d'organopolysiloxane réticulé élastomère enrobées de résine de silicone, notamment de résine silsesquioxane, comme décrit par exemple dans le brevet US 5 538 793. De telles poudres d'élastomère sont vendues sous les dénominations « KSP-100 », « KSP-101 », « KSP-102 », « KSP-103 », « KSP-104 », « KSP-105 » par la société SHIN ETSU, et
  - les poudres composites de talc/dioxyde de titane/alumine/silice comme celles vendues sous la dénomination Coverleaf AR-80 par la société Catalyst & chemicals,

  et leurs mélanges
- des composés absorbant et/ou adsorbant le sébum tels que décrit dans la même demande de brevet FR 2 869 796. On peut citer notamment :

  - les poudres de silice, comme par exemple les microsphères de silice poreuses vendues sous la dénomination "SILICA BEADS SB-700" commercialisées par la société MYOSHI, les "SUNSPHERE® H51", "SUNSPHERE® H33" , "SUNSPHERE® H53" commercialisées par la société ASAHI GLASS ; les microsphères de silice amorphe enrobées de polydiméthylsiloxane vendues sous la dénomination "SA SUNSPHERE® H-33" et "SA SUNSPHERE® H-53" commercialisées par la société ASAHI GLASS ;
  - les poudres de silicates mixtes amorphes, notamment d'aluminium et de magnésium, comme par exemple celle commercialisée sous la dénomination « NEUSILIN UFL2 » par la société Sumitomo.
  - les poudres de polyamides (nylon®), comme par exemple "l'ORGASOL® 4000" commercialisé par la société ATOCHEM, et
  - les poudres de polymères acryliques, notamment de polyméthacrylate de méthyle, comme par exemple le "COVABEAD® LH85" commercialisé par la société WACKHERR; de polyméthacrylate de méthyle/diméthacrylate d'éthylène glycol, comme par exemple le "DOW CORNING 5640 MICROSPONGE® SKIN OIL ADSORBER" commercialisé par la société DOW CORNING, ou le "GANZPEARL® GMP-0820" commercialisé par la société GANZ CHEMICAL ; de polyméthacrylate d'allyle/diméthacrylate d'éthylène glycol, comme par exemple le "POLY-PORE® L200" ou le "POLY-PORE® E200" commercialisés par la société AMCOL ; de copolymère diméthacrylate d'éthylène glycol/méthacrylate de lauryle, comme par exemple le "POLYTRAP® 6603" commercialisé de la société DOW CORNING ;
- les particules de silicate, telle que la silicate d'alumine ; ;
- les particules de silicates mixtes, telles que :

    o les particules de silicate d'aluminium et de magnésium, telles que la saponite ou silicate de magnésium et d'aluminium hydraté avec un sulfate de sodium commercialisée sous la dénomination commerciale Sumecton® par la société Kunimine ;
    o le complexe silicate de magnésium, hydroxyéthylcellulose, huile de cumin noir, huile de courge et phospholipides ou Matipure® de Lucas Meyer.

et leurs mélanges.

Comme agents matifiants préférés, on pourra utiliser selon l'invention un extrait de graines de potiron, un amidon de riz ou de mais, la kaolinite, des silices, le talc, les poudres de polyamides, les poudres de polyethylènes, les poudres de copolymères acryliques, les microsphères de copolymères acryliques expansées, les microbilles de résines de silicones, les particules de silicate mixte et leurs mélanges.

Charges abrasives ou agents exfoliants

Comme agents exfoliants utilisables dans des compositions rincées selon l'invention, on peut citer par exemple des particules exfoliantes ou gommantes d'origine minérale, végétale ou organique. Ainsi, on peut utiliser par exemple des billes ou de la poudre de polyéthylène, de la poudre de nylon, de la poudre de polychlorure de vinyle, de la pierre ponce, des broyats de noyaux d'abricots ou de coques de noix, de la sciure de bois, des billes de verre, l'alumine, et leurs mélanges.

On peut citer aussi l'Exfogreen® de Solabia (extrait de bambou), des extraits d'akenes de fraises (Akenes de fraise de Greentech), de la poudre de noyau de pêche, la poudre de noyau d'abricot, et enfin dans le domaine des poudres végétales à effet abrasif, citons la poudre de noyaux d'airelles (cranberry).

Comme charges abrasives ou agents exfoliants préférés selon l'invention, on citera la poudre de noyaux de pêche, la poudre de noyaux d'abricot, la poudre de noyaux d'airelles, les extraits d'akènes de fraise, les extraits de bambou.

Par l'expression « actif additionnel de soin des peaux grasses », on entend, dans le cadre de la présente invention, un composé qui a par lui-même, c'est-à-dire ne nécessitant pas l'intervention d'un agent extérieur pour l'activer, une activité biologique qui peut être en particulier :

- une activité desquamante (qui permet l'ouverture des comédons), et/ou
- une activité anti-microbienne (notamment sur *P. acnes),* et/ou
- une activité apaisante ou anti-inflammatoire, et/ou
- une activité sébo-régulatrice, et/ou
- une activité anti-oxydante (qui empêche l'oxydation du squalène et la formation des comédons)
- une activité cicatrisante ;
- une activité astringente.

Comme exemples d'actifs associés aux dérivés diphényl-méthane hydroxylés utilisables dans les compositions de l'invention, on pourra donc notamment citer les agents desquamants, les agents antimicrobiens, les agents apaisants, les agents anti-inflammatoires, les agents sébo-régulateurs, les agents antioxydants et leurs mélanges.

De préférence la composition selon l'invention comprendra comme actif additionnel au moins un agent sébo-régulateur.
Selon un mode particulier, la composition pourra comprendre en outre éventuellement un agent antimicrobien.
De façon encore préférée elle pourra comprendre en plus un agent desquamant.
Et pour augmenter encore l'efficacité et/ou la tolérance desdites compositions, on pourra rajouter en outre des agents apaisants ou anti-inflammatoires, des agents antioxydants, des agents cicatrisants, des agents astringents, et leurs mélanges.

Comme exemples de composés, pour chaque classe, on pourra citer notamment :

Agents sébo-régulateurs ou anti-séborrhéiques

Par agents "séborégulateurs ou anti-séborrhéiques ", on entend notamment des agents capables de réguler l'activité des glandes sébacées.

On peut citer notamment :

- l'acide rétinoïque, le peroxyde de benzoyle, le soufre, la vitamine B6 (ou pyridoxine ), le chlorure de sélénium, la criste marine ;
- les mélanges d'extrait de canelle, de thé et d'octanoylglycine tel que le Sepicontrol A5 TEA de chez Seppic ;
- le mélange de capryloyl glycine, de sarcosine et d'extrait de cinnamomum zeylanicum commercialisé notamment par la société SEPPIC sous la dénomination commerciale Sepicontrol A5® ;
- les sels de zinc tels que le gluconate de zinc, le pyrrolidone carboxylate de zinc (ou pidolate de zinc), le lactate de zinc, l'aspartate de zinc, le carboxylate de zinc, le salicylate de zinc, le cystéate de zinc ;
- les sels de cuivre, en particulier le pidolate de cuivre ;
- des extraits de végétaux des espèces Arnica montana, Cinchona succirubra, Eugenia caryophyllata, Humulus lupulus, Hypericum perforatum, Mentha piperita, Rosmarinus officinalis, Salvia oficinalis et Thymus vulgaris, tous commercialisés par exemple par la société MARUZEN ;
- les extraits de reine des prés (spiraea ulamaria) tel que celui vendu sous la dénomination Sébonormine® par la société Silab ;
- les extraits d'algue laminaria saccharina tel que celui vendu sous la dénomination Phlorogine® par la société Biotechmarine ;
- les extraits de sucre de canne, tel que celui commercialisé sous la dénomination Policasonol® par la société Sabinsa ;
- les mélanges d'extraits de racines de pimprenelle (sanguisorba officinalis/poterium officinale), de rhizomes de gingembre (zingiber officinalis) et d'écorce de cannelier (cinnamomum cassia) tel que celui vendu sous la dénomination Sebustop® par la société Solabia ;
- les extraits de graines de lin tel que celui vendu sous la dénomination Linumine® par la société Lucas Meyer ;
- les extraits de Phellodendron tels que ceux vendu sous la dénomination Phellodendron extract BG par la société Maruzen ou Oubaku liquid B par la société Ichimaru Pharcos ;
- les mélanges d'huile d'argan, d'extrait de serenoa serrulata (saw palmetto) et d'extrait de graines de sésame tel que celui vendu sous la dénomination Regu SEB® par la société Pentapharm ;
- les mélanges d'extraits d'epilobe, de terminalia chebula, de capucine et de zinc biodisponible (microalgues) tel que celui vendu sous la denomination Seborilys® par la société green tech ;
- les extraits de Pygeum afrianum tel que celui vendu sous la dénomination Pygeum afrianum sterolic lipid extract par la société Euromed ;
- les extraits de serenoa serrulata tels que ceux vendus sous les dénomination Viapure Sabal par la société Actives International, ou ceux vendus par la société Euromed ;
- les mélanges d'extraits de plantain, de berberis aquifolium et de salicylate de sodium tels que celui vendu sous la dénomination Seboclear® par la société Rahn ;
- l'extrait de clou de girofle tel que celui vendu sous la dénomination Clove extract Powder par la société Maruzen ;
- l'huile d'argan telle que celle vendue sous la dénomination Lipofructyl® par les Laboratoires Sérobiologiques ;
- les filtrats de protéine lactique tels que celui vendu sous la dénomination Normaseb® par la société Sederma ;
- les extraits d'algue laminaria, tel que celui vendu sous la dénomination Laminarghane® par la société Biotechmarine ;
- les oligosaccharides d'algue laminaria digitata tel que celui vendu sous la dénomination Phycosaccharide AC par la société Codif ;
- l'huile de schiste sulfonée, telle que celle vendue sous la dénomination Ichtyol Pale par la société Ichthyol ;
- les extraits d'ulmaire (spiraea ulmaria) tels que celui vendu sous la dénomination Cytobiol Ulmaire par la société Libiol ;
- l'acide sébacique, notamment vendu sous la forme d'un gel de polyacrylate de sodium sous la dénomination Sebosoft par la société Sederma ;
- les glucomannanes extraits de tubercule de konjac et modifié par des chaînes alkylsulfonates tel que celui vendu sous la dénomination Biopol Beta par la société Arch Chemical ;
- les extraits de Sophora angustifolia, tels que ceux vendus sous la dénomination Sophora powder ou Sophora extract par la société Bioland ;
- les extraits de cinchona succirubra bark tel que celui vendu sous la dénomination le Red bark HS par la société Alban Muller ;
- les extraits de quillaja saponaria tel que celui vendu sous la dénomination Panama wood HS par la société Alban Muller ;
- la glycine greffée sur chaîne undécylénique , telle que celle vendue sous la dénomination Lipacide UG OR par la

société Seppic ;

- le mélange d'acide oléanolique et d'acide nordihydroguaïarétique , tel que celui vendus sous la forme d'un gel sous la dénomination AC.Net par la société Sederma ;
- l'acide phthalimidoperoxyhexanoïque ;
- le citrate de trialkyle($C_{12}$-$C_{13}$) vendu sous la dénomination COSMACOL® ECI par la société Sasol ; le citrate de trialkyle($C_{14}$-$C_{15}$) vendu sous la dénomination COSMACOL® ECL par la société Sasol ;
- l'acide 10-hydroxydécanoïque, et notamment les mélanges d'acide 10-hydroxydécanoïque, d'acide sébacique et de 1,10-décandiol tels que celui vendu sous la dénomination Acnacidol® BG par la société Vincience ;
- les activateurs PPAR-$\gamma$ spécifiques comme ceux décrits dans la demande WO 2005/053632 ;
- des extraits de plantes du genre Silybum ;
- des sapogénines ou extraits végétaux en contenant, en particulier les extraits de Dioscorées riches en diosgénine ; et
- des extraits d'Eugenia caryophyllata contenant de l'eugénol et du glucoside d'eugényle, et leurs mélanges.

Comme agents séborégulateurs préférés utilisables selon l'invention, on citera :

- la criste marine ;
- les mélanges d'extrait de canelle, de thé et d'octanoylglycine tel que le Sepicontrol A5 TEA de chez Seppic ;
- le mélange de capryloyl glycine, de sarcosine et d'extrait de cinnamomum zeylanicum commercialisé notamment par la société SEPPIC sous la dénomination commerciale Sepicontrol A5® ;
- les sels de zinc tels que le gluconate de zinc, le pyrrolidone carboxylate de zinc (ou pidolate de zinc), le lactate de zinc, l'aspartate de zinc, le carboxylate de zinc, le salicylate de zinc, le cystéate de zinc ;
- les sels de cuivre, en particulier le pidolate de cuivre ;
- les extraits de reine des prés (spiraea ulamaria) tel que celui vendu sous la dénomination Sébonormine® par la société Silab ;
- les extraits d'algue laminaria saccharina tel que celui vendu sous la dénomination Phlorogine® par la société Biotechmarine ;
- les mélanges d'extraits de racines de pimprenelle (sanguisorba officinalis/poterium officinale), de rhizomes de gingembre (zingiber officinalis) et d'écorce de cannelier (cinnamomum cassia) tel que celui vendu sous la dénomination Sebustop® par la société Solabia ;
- les sapogénines ou extraits végétaux en contenant, en particulier les extraits de Dioscorées riches en diosgénine, et leurs mélanges.

On peut encore citer les antitranspirants, tels que : les sels d'aluminium et/ou de zirconium ; les complexes d'hydroxychlorure de zirconium et d'hydroxychlorure d'aluminium avec un acide aminé tels que ceux décrits dans le brevet US-3792068 communément connus sous l'appellation "ZAG complexes". De tels complexes sont généralement connus sous l'appellation ZAG (lorsque l'acide aminé est la Glycine). Les complexes ZAG présentent d'ordinaire un quotient Al/Zr allant d'environ 1,67 à 12,5 et un quotient Métal/Cl allant d'environ 0,73 à 1,93. Parmi ces produits on peut citer l'aluminium zirconium octachlorohydrex GLY, l'aluminium zirconium pentachlorohydrex GLY, l'aluminium zirconium tetrachlorohydrate GLY et l'aluminium zirconium trichlorohydrate-GLY.
Parmi les sels d'aluminium, on peut citer le chlorhydrate d'aluminium, l'aluminium chlorohydrex, l'aluminium chlorohydrex PEG, l'aluminium chlorohydrex PG, l'aluminium dichlorohydrate, l'aluminium dichlorohydrex PEG, l'aluminium dichlorohydrex PG, l'aluminium sesquichlorohydrate, l'aluminium sesquichlorohydrex PEG, l'aluminium sesquichlorohydrex PG, les sels d'alun, l'aluminium sulfate, l'aluminium zirconium octachlorohydrate, l'aluminium zirconium pentachlorohydrate, l'aluminium zirconium tetrachlorohydrate, l'aluminium zirconium trichlorohydrate et plus particulièrement l'aluminium chlorhydrate commercialisé par la société REHEIS sous la dénomination MICRODRY ALUMINUM CHLOROHYDRATE ou par la société GUILINI CHEMIE sous la dénomination ALOXICOLL PF 40. Des sels d'aluminium et de zirconium sont par exemple celui commercialisé par la société REHEIS sous la dénomination REACH AZP-908-SUF , des sel d'aluminium « activés » par exemple celui commercialisé par la société REHEIS sous la dénomination REACH 103 ou par la société WESTWOOD sous la dénomination WESTCHLOR 200.

Parmi les autres actifs déodorants, on peut citer également les sels de zinc comme le salicylate de zinc, le sulfate de zinc, le chlorure de zinc, le lactate de zinc, le phénolsulfonate de zinc ; la chlorhexidine et les sels; le monocaprate de diglycérol, le monolaurate de diglycérol , monolaurate de glycérol ; les sels de polyhexaméthylène biguanide.

Agents anti-microbiens

Par agents anti-microbiens, on entend des agents ayant des effets sur la flore spécifique des peaux grasses, tels que par exemple le *P acnes.*
Ces effets peuvent être soit bactéricides, soit anti-adhésion bactérienne (prévient et/ou réduit l'adhésion des micro-organismes) soit agissant sur le biofilm des bactéries pour éviter leur multiplication.
On peut citer notamment les actifs et conservateurs à activité anti-microbienne cités dans la demande DE10324567, incorporée dans la présente invention par référence.
On peut citer également :

- un extrait de cône de houblon obtenu par extraction $CO_2$ supercritique tel que HOP CO2-TO extract® de Flavex Naturextrakte.,
- un extrait de Millepertius obtenu par extraction $CO_2$ supercritique tel ST John's Wort CO2-TO extract® de Flavex,
- l'acide asiatique,
- le mélange d'extraits de racines de scutellaria baicolensis, de paeonia suffruticosa et de glycyrrhiza glabra, comme dans le BMB - CF® de Naturogin,
- le sel de monoéthanolamine du 1-hydroxy-4-methyl 6-trimethylpentyl 2-pyridone (nom INCI : la piroctone olamine), notamment vendu sous la dénomination Octopirox® par la société Clariant ;,
- l'acide citrollique, l'acide spérillique (ou acide 4-isopropenylcyclohex-1-ènecarboxylique),
- le 2-éthyl hexyle éther de glycérol (nom INCI : l'ethylhexylglycerine) par exemple le Sensiva SC 50® De Shulke & Mayr,
- le caprylate/caprate de glyceryle (Capmul MCM® de ABITEC),
- le calcium sodium phosphosilicate comme Bioactive glasspowder r® de Schott,- les Actysse premier BG r® de Schott,
- les oxydes de silicium de Ciba,
- les particules à base d'argent, comme les Métashines ME 2025 PS® de la société Nippon Sheet Glass ,
- l'extrait d'arganier comme l'Argapure LS9710® de chez COGNIS ;
- les extraits de busserole comme la Gatuline equalizing de Gattefossé ou "Melfade-J" par la société Pentapharm,
- l'acide 10-hydroxy-2 decanoique comme l'Acnacidol P de Vincience, l'ursolate de sodium, l'acide azélaique, le di-iodo-methyl P tolylsulfone ou l'Amical Flowable® de Angus, la la poudre de Malachite de Maprecos, l'oxyde de zinc tel que le Zincare® de Elementis GMBH, l'acide octadécènedioïque tel que l'arlatone dioic DCA® de Uniqema, l'acide phthalimidoperoxyhexanoique ou Eureco HC de Chemron Corporation ; l'acide ellagique ; le 2,4,4'-trichloro-2'-hydroxy diphényl éther (ou triclosan), la 1-(3',4'-dichlorophenyl)-3-(4'-chlorophenyl)urée (ou triclocarban), le 3,4,4'-trichlorocarbanilide, le 3',4',5'-trichlorosalicylanilide, le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopirox, le ciclopiroxolamine, l'acide undécylenique et ses sels, le peroxyde de benzoyle, l'acide 3-hydroxy benzoïque, l'acide 4-hydroxy benzoïque, l'acide phytique, la N-acétyl-L-cystéine, l'acide lipoïque, l'acide azélaïque et ses sels, l'acide arachidonique, le résorcinol, le 3,4,4'-trichlorocarbanalide, l'octopirox ou piroctone olamine, l'octoxyglycérine ou octoglycérine, l'octanoylglycine (Lipacid C8G® de Seppic), le caprylyl glycol, l'acide 10-hydroxy-2-décanoïque, le dichlorophenyl imidazol dioxolan et ses dérivés décrits dans la demande de brevet WO9318743, les dérivés de zinc et en particulier le pidolate de zinc (Zincidone® de Solabia), les dérivés de cuivre et en particulier le pidolate de cuivre (Cuivridone® de Solabia), l'acide salicylique et ses dérivés, l'iodopropynyl butylcarbamate, le 3,7,11-triméthyldodéca-2,5,10-triénol ou farnesol, les phytosphingosines ; le Sepicontrol® de Seppic, un extrait d'arganier comme l'Argapure LS9710®, le Sebosoft® de Sederma, les sels d'ammonium quaternaires comme les sels de cetyltrimethylammonium, les sels de cétylpyridinium , l'éthanol...et leurs mélanges.

Comme agents prévenant et/ou réduisant l'adhésion des micro-organismes, on peut citer notamment : le phytanetriol et ses dérivés tels que décrits dans la demande de brevet EP 1 529 523, les huiles végétales telles de l'huile de germe de blé, l'huile de calendula, l'huile de ricin, l'huile d'olive, l'huile d'avocat, l'huile d'amande douce, l'huile d'arachide, l'huile de jojoba, l'huile de sésame, l'huile d'amande d'abricot, l'huile de tournesol, l'huile de macadamia, décrites dans le brevet EP 1 133 979, ou encore d'autres corps gras tels que le cocoamphodiacétate disodique, le cocoate de glycéryle oxyéthyléné (7 OE), les Poloxamers, l'hexadécénylsuccinate 18, le palmitate d'octoxyglycéryle, le béhénate d'octoxyglycéryle, l'adipate de dioctyle , le PPG-15 stéaryl éther, le tartrate de di-alcools C12-C13 ramifiés décrits dans le brevet EP 1 129 694.
En particulier vis-à-vis de la propagation du *P acnes,* on peut citer le pentylène glycol, le nylon-66 (fibres de polyamides 66), l'huile de son de riz , l'alcool polyvinylique tel que le Celvol 540 PV alcohol® de chez Celanese Chemical ), l'huile de colza telle que l'Akorex L® de Karlshamns, les dérivés de fructose.
On peut également citer certains tensioactifs ayant un effet antimicrobien comme le cocoampho acétate de sodium ou diacetate disodium tel que le Miranol C2M CONC NP, les bétaines comme la cocoyl bétaine Genagen KB de Clariant, le lauryl ether sulfate de sodium comme l'Emal 270 D de Kao, le décyl glucoside comme le Plantacare 2000 UP, les malate de dialcools C12-13 ramifiés comme le Cosmacol EMI, les monoesters de propylène glycol comme monolaurate, monocaprylate, monocaprate de propylène glycol, le sodium lauroyl oat amino acid comme le Proteol

OAT, le lauryl dimethylamine betaine comme le Empigen BB/LS ainsi que les polyammonium quaternaires comme le Quaternium-24 ou Bardac 2050 de Lonza et ceux décrits dans le brevet L'OREAL FR 0 108 283.

Comme agents antimicrobiens préférés, on utilisera dans les compositions de l'invention un agent choisi parmi le caprylyl glycol, les dérivés de zinc dont le pidolate de zinc (Zincidone® de Solabia), les dérivés de cuivre dont le pidolate de cuivre (Cuivridone® de Solabia), l'octoglycérine ou octoxyglycérine, l'acide 10-hydroxy-2-décanoïque, et leurs mélanges.

Agents desquamants

Par "agent desquamant", on entend tout composé capable d'agir :

- soit directement sur la desquamation en favorisant l'exfoliation, tel que les $\beta$-hydroxyacides, en particulier l'acide salicylique et ses dérivés (dont l'acide n-octanoyl 5-salicylique) ; les $\alpha$-hydroxyacides, tels que les acides glycolique, citrique, lactique, tartrique, malique ou mandélique ; l'urée ; l'acide gentisique et ses dérivés ; les oligofucoses ; l'acide cinnamique ; l'extrait de Saphora japonica ; le resvératrol et certains dérivés d'acide jasmonique ;

- soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

Comme autres agents desquamants utilisables dans la composition selon l'invention, on peut citer les oligofructoses, l'EDTA et ses dérivés, les laminaria extract, le o-linoleyl-6D-glucose, le (3-hydroxy-2pentylcyclopentyl)acetic acid, le trilactate de glycérol, l'O-octanyl-6'-D-maltose, la S carboxyméthyl cystéine, les dérivés siliciés de salicylate comme dans le brevet EP 0 796 861, les oligofucase comme dans le brevet EP 0 218 200, les sels d'acide 5-acyl salicylique, des actifs ayant des effets sur la transglutaminase comme dans le brevet EP 0 899 330, l'acide jasmonique et dérivés comme dans les demandes de brevet EP 1 333 022 et EP 1 333 021 ; l'Exfolactive® de Silab (extrait de fleur de ficus opuntia indica), le Soypon O® de Kawaken fine chemicals (sodium cocoyl sarcosinate).

Comme agents desquamants préférés, on pourra citer les beta-hydroxyacides, tel que l'acide n-octanoyl 5-salicylique ; l'urée ; les acides glycolique, citrique, lactique, tartrique, malique ou mandélique ; l'acide (N-2 hydroxyéthylpiperazine-N-2-éthane) sulfonique (HEPES) ; l'extrait de Saphora japonica ; le miel ; le N-acétyl glucosamine ; le méthyl glycine diacétate de sodium, et leurs mélanges.

Encore plus préférentiellement on utilisera dans els compositions de l'invention un agent edsquamant choisi parmi l'acide n-octanoyl 5-salicylique ; l'urée ; l'acide (N-2 hydroxyéthylpiperazine-N-2-éthane) sulfonique (HEPES) ; l'extrait de Saphora japonica ; le miel ; le N-acétyl glucosamine ; le méthyl glycine diacétate de sodium, et leurs mélanges.

Agents apaisants ou anti-irritants

On peut citer notamment les agents apaisants ou anti-irritants cités dans les demandes WO2004/105736 et DE10324567, incorporées dans la présente invention par référence.

Comme agents apaisants particuliers utilisables dans la composition selon l'invention, on peut citer : les oligomères procyannidoliques, les vitamines E, C B5, B3, le sulfate de dextran, la caféine et ses dérivés, les triterpènes pentacycliques et les extraits de plantes les contenant, l'acide b-glycyrrhétinique et ses sels ou dérivés (le stearyl glycyrrhetate, l'acide 3-stéaroyloxy glycyrrhétique, l'acide glycyrrhétinique monoglucuronide) ainsi que les plantes en contenant (ex : Glycyrrhiza glabra), l'acide oléanolique et ses sels, l'acide ursolique et ses sels, l'acide boswellique et ses sels, l'acide bétulinique et ses sels, un extrait de Paeonia suffruticosa et/ou lactiflora, le salycilate de zinc, les phycosshacharides de la société Codif, un extrait de Laminaria saccharina, les extraits de Centella asiatica, l'huile de Canola, le bisabolol,le diesterphosphorique de vitamine E et C comme le Sepivital EPC® de Seppic, les extraits de camomille, l'allantoïne, les huiles insaturées en oméga 3 telles que les huiles de rosier musca, de cassis, d'Ecchium, de poisson, l'huile de calophilum, des extraits de plancton, la capryloyl glycine, un mélange d'extrait de fleur de nénuphar et de palmitoylproline tel que le Seppicalm VG® (nymphea alba et sodium palmitoylproline) de Seppic, un extrait de Pygeum, un extrait de Boswellia serrata, un extrait de Centipeda cunnighami tel que celui vendu sous la dénomination "Cehami PF®" par la société TRI-K Industries, un extrait d'Helianthus annus en particulier l'Hélioxine® de Silab, un extrait de Linum usitatissimum comme la Sensiline® de Silab, les tocotriénols, les extraits de Cola nitida, le piperonal, un extrait de clou de girofle, un extrait d'Epilobium angustifolium, l'aloe vera, un extrait de Bacopa moniera, les phytostérols, l'eau

de bleuet, l'eau de rose, le dextran comme dans Modulène® de Vincience, un extrait de menthe, en particulier de feuilles de menthe comme le Calmiskin® de Silab, les dérivés d'anis, les bactéries filamenteuse comme Vitreoscilla filiformis tel que décrit dans le brevet EP 761 204 et commercialisé par Chimex sous la dénomination Mexoryl SBG®" un extrait de rose comme l'Herbasol rose extract® de Cosmetochem le beurre de karité, un mélange de fraction de cire de graine d'orge obtenue par $CO_2$ supercritique, de beurre de karité et d'huile d'argan comme le Stimu-tex AS® de Pentapharm, les sels alcalino-terreux notamment le strontium, la niacinamide, un extrait fermenté d'Alteromonas commercialisé sous la dénomination ABYSSINE® par la société Atrium Biotechnologies ; les eaux thermales du bassin de Vichy, telles que les eaux provenant des sources Célestins, Chomel, Grande-Grille, Hôpital, Lucas et Parc, et de préférence l'eau de la source Lucas ; un extrait d'écorce d'Eperua falcata tel que celui commercialisé par la société COGNIS sous la dénomination Eperuline® ; un extrait de racine de Paeonia suffructicosa tel que celui commercialisé par la société Ichimaru Pharcos sous la dénomination Botanpi Liquid B® , et leurs mélanges.

Comme agents apaisants préférés, on utilisera un agent choisi parmi un extrait de rose, un extrait de clou de girofle, le dextran comme dans Modulène® de Vincience, un extrait de menthe comme le Calmiskin® de Silab, les bactéries filamenteuse comme Vitreoscilla filiformis tel que décrit dans le brevet EP 761 204 et commercialisé par Chimex sous la dénomination Mexoryl SBG®, un mélange d'un extrait de nymphea alba et sodium palmitoylproline comme le Seppicalm VG® de Seppic, des dérivés d'anis, un extrait de Paeonia suffruticosa et/ou lactiflora, et leurs mélanges.

Agents anti-inflammatoires

On peut citer notamment les agents anti-inflammatoires cités dans les demandes WO2004/105736 et DE10324567, incorporées dans la présente invention par référence.

Comme agents anti-inflammatoires particuliers utilisables selon l'invention, on peut citer la cortisone, l'hydrocortisone, l'indométhacine, la bétaméthasone, l'acide azéalique, l'acétominophène, le diclofénac, le propionate de clobetasol, l'acide folique ; un extrait d'écorce d'Eperua falcata tel que celui commercialisé par la société COGNIS sous la dénomination Eperuline® ; un extrait de racine de Paeonia suffructicosa tel que celui commercialisé par la société Ichimaru Pharcos sous la dénomination Botanpi Liquid B® ; et leurs mélanges.

Comme agent anti-inflammatoire préféré, on citera l'acide azélaique, l'acide folique, un extrait d'écorce d'Eperua falcata tel que celui commercialisé par la société COGNIS sous al dénomination Eperuline® ; un extrait de racine de Paeonia suffructicosa tel que celui commercialisé par la société Ichimaru Pharcos sous la dénomination Botanpi Liquid B® ; et leurs mélanges.

Agents antioxydants

On entend les agents une activité anti-oxydante (qui empêchent l'oxydation du squalène et la formation des comédons).

On peut citer notamment le tocophérol et ses esters, en particulier l'acétate de tocophérol ; le BHT et le BHA. On peut également citer les polyphénols, l'acide tannique, l'époigallocathéchines et les extraits naturels en contenant, les anthocyanes, les extraits de romarin, les extraits de feuilles d'olivier, le thé vert, le resvératrol et ses dérivés, le Pycnogénol, l'ergothinéine, la N acétylcystéine, la biotine, les chélatants, l'idébénone, des extraits végétaux come le Pronalen Bioprotect TM de la société Provital, les antiradicalaires comme la vitamine E, le co enzyme Q10, les bioflavonoides, les SOD, le phytantriol, les lignanes, la mélatonine, les pidolates, le gluthation.

Agents cicatrisants

Comme exemples d'agents cicatrisants, on peut citer notamment :

l'allantoine, l'urée, l'huile de germe de blé, certains acides aminés comme l'hydroxyproline, l'arginine, la sérine, et aussi des extraits de lys blanc (ex : le Phytélène Lys 37EG 16295 de Indena), un extrait de levures comme le cicatrisant LS 7225B de LS (Cognis), l'huile de tamanu, l'extrait de saccharomyces cerevisiae ou Biodynes TRF® de Arch Chemical, les extraits d'avoine, le chitosane et dérivés, les extraits de carotte, l'extrait d'artemia ou GP4G de Vincience, l'acexamate de sodium, des extraits de lavandin, des extraits de miel ou de propolis, l'acide ximeninique et ses sels tel que acido ximeninico de Indena, l'huile de rosa rugosa, des extraits de souci comme le Souci Ami Liposolible d'Alban Muller, des extraits de prêle, les extraits d'écorce de citron comme l'herbasol citron de Cosmetochem, des extraits d'helichryse, des béta-glucan et dérivés, du beurre de karité et ses fractions purifiées, les exopolysaccharides modifiés et les Polyaminosaccharides alkylsulfonnés, des extraits de millefeuilles, et l'acide folique.

Comme agents cicatrisants préférés selon l'invention, on utilisera , la sérine, l'acide folique, l'huile de tamanu,

l'acexamate de sodium, des extraits de miel, des extraits de prêle, des extraits d'helichryse, et leurs mélanges.

Agents astringents

Par 'agents astringents', on entend selon l'invention des agents permettant de lutter contre la dilatation des follicules sébacés.

Comme agents astringents utilisables dans la composition selon l'invention, on peut citer des extraits de pulpe de champignon (polyporus officinalis) comme le Laricyl LS8865® de Cognis, des extraits de Terminalia catappa et sambucus nigra comme le Phytofirm LS9120® de Cognis , des extraits de noix de galle comme le Tanlex VE/VB® de Ichimaru Pharcos, la laponite, les sels d'aluminium, l'hydroxychlorure d'aluminium, les extraits de centella (ex Plantactiv centella de Cognis), le chlorure de dicétyl diméthyl ammonium comme le Varisoft 432 CG® de Degussa, les extraits de marron d'inde, les extraits de mauve, d'Hammamelis, des extraits d'amandes douces, de racines de guimauve et de graines de lin comme l'Almondermin LS 3380® de Cognis, les extraits de bardane, les extraits d'ortie, les extraits de bouleau, les extraits de prêle, les extraits de camomille comme ceux vendus sous la dénomination l'Extrapone 9 specia®l de Symrise, les extraits de scutellaria, les extrais d'Ulmaire (ex Cytobiol Ulmaire de Libiol), un mélange d'extraits de gingembre blanc, de prêle, d'ortie, de romarin, de yucca comme l'Herb extract B1348® de Bell flavors & fragrances, les extraits d'acacia, d'orme, de saule blanc, de canelle, de bouleau, de reine des prés, les sapogénines de panama, le phenolsulfonate de zinc de Interchemical, des extraits de gentiane, de concombre, de noyer, le mélange d'extraits de Ratanhia, de pamplemousse, de grindelia et de galle de chêne comme Epilami® de Alban Muller.

Comme agents astringents préférés selon l'invention, on utilisera les extrais de scutellaria, les extraits d'ulmaire, les extraits de reine des près, les extraits de gentiane, les extraits de bardane et leurs mélanges.

**b) actifs et/ou ingrédients additionnels pour lutter contre les signes du vieillissement cutané**

Une composition cosmétique anti-âge selon l'invention comprendra avantageusement en association avec ledit dérivé diphényl-méthane hydroxylé de formule (I) ou (II), un ingrédient et/ou un actif additionnel choisi parmi : les charges, les azurants optiques, les agents de fluorescence, les agents stimulant la synthèse de macromolécules dermiques et/ou épidermiques et/ou empêchant leur dégradation, les agents stimulant la prolifération des fibroblastes ou des kératinocytes et/ou la différenciation des kératinocytes, les agents favorisant la fonction barrière cutanée, les agents favorisant la maturation de l'enveloppe cornée, les inhibiteurs de NO-synthases, les antagonistes des récepteurs périphériques des benzodiazépines, les agents augmentant l'activité de la glande sébacée, les agents anti-glycation, les agents dermorelaxants ou dermodécontractants, les agents favorisant la microcirculation cutanée, les agents stimulant le métabolisme énergétique des cellules, les agents tenseurs et leurs mélanges.

Selon un mode particulier, ladite composition comprend, en association avec ledit dérivé diphényl-méthane hydroxylé de formule (I) ou (II), au moins un ingrédient additionnel favorisant la solubilisation et/ou la stabilisation dudit dérivé et au moins un ingrédient et/ou actif additionnel choisi parmi : les charges, les azurants optiques, les agents de fluorescence, les agents stimulant la synthèse de macromolécules dermiques et/ou épidermiques et/ou empêchant leur dégradation, les agents stimulant la prolifération des fibroblastes ou des kératinocytes et/ou la différenciation des kératinocytes, les agents favorisnt la fonction barrière cutanée, les agents favorisant la maturation de l'enveloppe cornée, les inhibiteurs de NO-synthases, les antagonistes des récepteurs périphériques des benzodiazépines, les agents augmentant l'activité de la glande sébacée, les agents anti-glycation, les agents dermorelaxants ou dermodécontractants, les agents favorisant la microcirculation cutanée, les agents stimulant le métabolisme énergétique des cellules, les agents tenseurs et leurs mélanges.

En particulier, la composition selon l'invention comprend au moins un dérivé diphényl méthane hydroxylé de formule (I) ou (II), au moins un ingrédient favorisant la solubilisation dudit dérivé et au moins un ingrédient et/ou actif additionnel choisi parmi :

les charges, les azurants optiques, les agents de fluorescence, les agents stimulant la synthèse de macromolécules dermiques et/ou épidermiques et/ou empêchant leur dégradation, les agents stimulant la prolifération des fibroblastes ou des kératinocytes et/ou la différenciation des kératinocytes, les agents favorisnt la fonction barrière cutanée, les agents favorisant la maturation de l'enveloppe cornée, les inhibiteurs de NO-synthases, les antagonistes des récepteurs périphériques des benzodiazépines, les agents augmentant l'activité de la glande sébacée, les agents anti-glycation, les agents dermorelaxants ou dermodécontractants, les agents favorisant la microcirculation cutanée, les agents stimulant le métabolisme énergétique des cellules, les agents tenseurs et leurs mélanges.

Les ingrédients de solubilisation préférés sont décrits précédemment. En particulier, il s'agira :

- d'un dérivé lipophile d'acide aminé, en particulier le N-lauroylsarcosinate d'isopropyle commercialisé notamment

par AJINOMOTO sous la dénomination ELDEW SL 205 ;

- d'un éther d'isosorbide, en particulier le dimethyl isosorbide commercialisé sous la dénomination ARLASOLVE DMI par la société UNIQEMA;
- d'un ester d'alcool caprylique, en particulier, le dicaprylyl carbonate commercialisé sous la dénomination CETIOL CC par la société COGNIS ;
- d'un dérivé de l'acide cinnamique, en le p-méthoxycinnamate de 2-éthylhexyle, ou l'octylmethoxycinnamate vendu notamment sous le nom commercial « PARSOL MCX » par la Société GIVAUDAN (DSM Nutritional Products) ;
- de l'octyl-2-dodécanol commercialisé sous la dénomination EUTANOL G par la société COGNIS ou sous la dénomination ISOFOL 20 N/F par la société SASOL ;

ou leurs mélanges.

Des exemples de tels ingrédients et/ou actifs additionnels sont décrits ci-après.

<u>1. Les agents de camouflage, en particulier les charges</u>

Par agents de camouflage, on entend les charges, les azurants optiques, les agents de fluorescence et leurs mélanges.

On entend par charge tout matériau susceptible de modifier les irrégularités du microrelief cutané, en particulier les rides et ridules, par ses propriétés physiques intrinsèques et de la masquer. On parle aussi de charges à effet floutteur ou charge 'soft focus'.

En tant que charge, on peut donner à titre d'exemple :

o Les microparticules poreuse de silice comme par exemple les Silica Beads SB 150 et SB 700 de Myochi de taille moyenne de 5μm et les SUNSPHERES série H d'Asahi Glass comme les H33, H51 de taille respectivement de 3.5 et 5 μm.

o Les particules hémisphériques creuses de silicones comme les séries NLK dont le NLK 500, NLK 506 et NLK 510 et NLK 523 de Takemoto Oil and Fat

o Les poudres de résine de silicone comme par exemple les SILICON Resin Tospearl 145 A DE GE silicone de taille moyenne de 4.5μm.

o Les poudres de copolymères acryliques, notamment de poly(meth)acrylate de méthyle comme par exemple les particules PMMA Jurimer MBI de Nihon Junyoki de taille moyenne de 8μm, les sphères creuses de PMMA vendues sous la dénomination COVABEAD LH 85 par la société Wackherr et les microsphères de vinylidène/acrylonitrile/méthacrylates de méthylène expansées vendues sous la dénomination Expancel

o Les poudres de cires comme les particules Paraffin wax microase 114S de Micropowders de taille moyenne de 7μm

o Les poudres de polyéthylènes notamment comprenant au moins un copolymère éthylène/acide acrylique comme par exemple les FLOBEADS EA 209 E de Sumimoto de taille moyenne de 10μm.

o Les poudres d'organopolysiloxanes élastomériques réticulées enrobées de résine de silicone notamment de silsesquioxane sous la dénomination KSP 100, KSP 101, KSP 102, KSP 103, KSP 104 et KSP 105 par la société Shin Etsu

o Les poudres composites de talc/dioxyde de titane/alumine/silice comme par exemple les Cverleaf AR 80 de la société Catalyst & Chemical.

o On peut aussi citer: le talc, le mica, le kaolin, la lauryl glycine, les poudres d'amidon réticulés par l'anhydride octéanyl succinate, le nitrure de bore, les poudres de polytétrafluoroéthylène, le carbonate de calcium précipité, le carbonate de l'hydrocarbonate de magnésium, le sulfate de baryum, l'hydroxyapatite, le silicate de calcium, le dioxyde de cérium et les microcapsules de verre ou de céramiques.

o des fibres, telles que des fibres de soie, de coton, de laine, de lin, de cellulose extraites notamment du bois, des légumes ou des algues, de polyamide (Nylon®), de cellulose modifiée, de poly-p-phénylène téréphtamide, en acrylique, de polyoléfine, de verre, de silice, d'aramide, de carbone, de Téflon®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères, les fibres synthétiques résorbables, et leurs mélanges décrites dans la demande de brevet L'OREAL EP 1 151 742.

Les charges sont notamment choisies parmi des microparticules poreuse de silice, des particules hémisphériques creuses de silicones, despoudres de résine de silicone, des poudres de copolymères acryliques, des poudres de polyéthylènes, des poudres d'organopolysiloxanes élastomériques réticulées enrobées de résine de silicone, des poudres composites de talc/dioxyde de titane/alumine/silice, le carbonate de calcium précipité, le carbonate de l'hydrocarbonate de magnésium, le sulfate de baryum, l'hydroxyapatite, le silicate de calcium, le dioxyde de cérium et les microcapsules de verre ou de céramiques, les fibres de soie, de coton, et leurs mélanges.

Une des charges préférées selon l'invention est l'hydroxyapatite.

La concentration de ces charges est généralement comprise entre 0.1 et 40 % en poids par rapport au poids total de la composition, de préférence de de 3,5 à 40% en poids,et encore plus préférentiellement de 5 à 15% en poids, par rapport au poids total de la composition.

Par charge « soft-focus », on entend une charge qui en plus donne de la transparence au teint et un effet flou. De préférence, les charges «soft-focus» ont une taille moyenne des particules inférieure ou égale à 15 microns. Ces particules peuvent être de toutes formes et en particulier être sphériques ou non sphériques. De préférence encore, ces charges sont non sphériques.

Les charges « soft-focus » peuvent être choisies parmi les poudres de silice et silicates, notamment d'alumine, les poudres de type polyméthyl méthacrylate (PMMA), le talc, les composites silice/$TiO_2$ ou silice/oxyde de zinc, les poudres de polyéthylène, les poudres d'amidon, les poudres de polyamides, les poudres de copolymères styrène/acrylique, les élastomères de silicone, et leurs mélanges.

En particulier, on peut citer le talc de taille moyenne en nombre inférieure ou égale à 3 microns, par exemple du talc de taille moyenne en nombre de 1,8 micron et notamment celui vendu sous la dénomination commerciale Talc P3® par la société Nippon Talc, la poudre de Nylon® 12, notamment celle vendue sous le dénomination Orgasol 2002 Extra D Nat Cos® par la société Atochem, les particules de silice traitées en surface par une cire minérale 1 à 2 % (nom INCI : hydrated silica (and) paraffin) telles que celles commercialisées par la société Degussa, les microsphères de silice amorphe, telles que celles vendues sous la dénomination Sunsphère par exemple de référence H-53 par la société Asahi Glass, et les micro-billes de silice telles que celles vendues sous la dénomination SB-700® ou SB-150® par la société Miyoshi, cette liste n'étant pas limitative.

La charge à effet de flou peut être présente dans la composition cosmétique à effet de flou en une teneur allant de 0,1 à 20 % en poids et notamment allant de 1 % à 12 % en poids par rapport au poids total de la composition, notamment entre 5 et 10 %, par exemple de l'ordre de 8 %.

On entend par agent fluorescent une substance qui, sous l'effet de rayons ultraviolet et/ou de la lumière visible, ré-émet dans le visible la portion de lumière qu'elle a absorbé sous la même couleur que celle qu'elle reflète naturellement. La couleur réfléchie naturellement est ainsi renforcée par la couleur ré émise et apparaît extrêmement brillante.

On peut citer par exemple les résines colorées de polyamide et/ou de formaldéhyde/benzoguanamine et/ou de melamine/formaldéhyde/sulfonamide, parmi les co-condensats aminotriazine/formaldéhyde/sulfonamide colorés et/ou parmi les paillettes polyester métallisées et/ou leurs mélanges. Ces pigments fluorescents peuvent aussi se présenter sous la forme de dispersions aqueuses de pigments fluorescents.

On peut encore citer le co-condensat aminotriazine/formaldehyde/sulfonamide fluorescent coloré en rose de taille moyenne des particules 3-4 microns vendu sous la dénomination commerciale « Fiesta Astral Pink FEX-1 » et le co-condensat aminotriazine/formaldehyde/sulfonamide fluorescent coloré en bleu de taille moyenne des particules 3-4,5 microns vendu sous la dénomination commerciale « Fiesta Comet Blue FTX-60 » par la société Swada ou encore la résine benzoguanamine/formaldéhyde recouverte de résine formaldéhyde/urée et colorée en jaune vendue sous la dénomination commerciale « FB-205 Yellow » et la résine benzoguanamine/formaldéhyde recouverte de résine formaldéhyde/urée et colorée en rouge vendue sous la dénomination commerciale « FB-400 Orange Red » par la société UK SEUNG CHEMICAL, la résine polyamide colorée en orange vendue sous la dénomination commerciale « Flare 911 Orange 4 » par la société Sterling Industrial Colors.

Les substances fluorescentes sont de préférence présentes dans la composition, à une teneur allant de 0,1 à 20%, de préférence de 0,1 à 15%, de préférence encore de 0,5 à 3% en poids, par rapport au poids total de la composition.

Lorsque les substances fluorescentes organiques sont blanches, on les appelle également des azurants optiques.

L'azurant optique a pour effet d'intensifier l'éclat et aviver les teintes des compositions cosmétiques les comprenant à l'application sur la peau.

Parmi les azurants optiques, on peut plus particulièrement citer les dérivés du stilbène, en particulier les polystyrylstilbènes et les triazinstilbènes, les dérivés coumariniques, en particulier les hydroxycoumarines et les aminocoumarines, les dérivés oxazole, benzoxazole, imidazole, triazole, pyrazoline, les dérivés du pyrène et les dérivés de porphyrine et/ou leurs mélanges.

De tels composés sont par exemple disponibles sous les dénominations commerciales Tinopal SOP.□et Uvitex OB.□ auprès de la société CIBA GEIGY.

Les azurants optiques préférentiellement utilisés sont le 4,4'-bis[(4,6-dianilino-1,3,5-triazin-2-yl)amino]stilbène-2,2'-disulfonate de sodium, le 2,5 thiophène di-yl bis(5 ter-butyl-1,3 benzoxazole), le di-styryl-4,4' biphényle sulfonate disodique et/ou leurs mélanges.

<u>2. Agents stimulant la synthèse de macromolécules dermiques et/ou épidermiques et/ou empêchant leur dégradation :</u>

Parmi les actifs stimulant les macromolécules du derme ou empêchant leur dégradation, on peut citer ceux

qui agissent :

- soit sur la synthèse du collagène, tels que les extraits de Centella asiatica, les asiaticosides et dérivés ; l'acide ascorbique ou vitamine C et ses dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; les peptides de riz tel que le Nutripeptide® de SILAB, le méthylsilanol mannuronate tel que l'Algisium C® commercialisé par Exsymol ; l'acide folique ; et un extrait de Medicago sativa (alfafa) tel que celui commercialisé par SILBA sous la dénomination Vitanol®; un extrait peptidique de noisette tel que celui commercialisé par la société Solabia sous la dénomination Nuteline C®; et l'arginineet les hormones végétales telles que les auxines et les lignanes ;

- soit sur l'inhibition de la dégradation du collagène, en particulier des agents agissant sur l'inhibition des métalloprotéinases (MMP) telles que plus particulièrement les MMP 1, 2, 3, 9 . On peut citer : les rétinoïdes et dérivés, les extraits de medicago sativa tels que le Vitanol® de Silab, un extrait d'aphanizomenon flos-aquae (cyanophycée) commercialisée sous la dénomination Lanablue® par Atrium Biotechnologies, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon ou de sauge ; la DIPALMITOYL HYDROXYPROLINE commercialisé par Seppic sous le nom SEPILIFT DPHP® : Baccharis genistelloide ou Baccharine commercialisé par SILAB, un extrait de moringa tel que l'Arganyl LS 9781® de Cognis ; l'extrait de sauge décrit dans la demande FR-A-2812544 de la famille des labiées (salvia officinalis de la société Flacksmann), l'extrait de Rhododendron, le blueberry extract, un extrait de vaccinium myrtillus tel que ceux décrits dans la demande FR-A-2814950..

- soit sur la synthèse de molécules appartenent à la famille des élastines (élastine et fibrilline), tels que : le retinol et dérivés ; l'extrait de *Saccharomyces Cerivisiae* commercialisé par la société LSN sous la dénomination commerciale Cytovitin® ; et l'extrait d'algue *Macrocystis pyrifera* commercialisé par la société SECMA sous la dénomination commerciale Kelpadelie® ; un extrait peptidique de noisette tel que celui commercialisé par la société Solabia sous la dénomination Nuteline C®.

- soit sur l'inhibition de la dégradation de l'élastine tels que l'extrait peptidique de graines de *Pisum sativum* commercialisé par la société LSN sous la dénomination commerciale Parelastyl® ; les héparinoïdes ; et les composés N-acylaminoamides décrits dans la demande WO 01/94381 tels que l'acide 12-[acétyl-(3-trifluorométhyl-phényl)-amino]-3-méthyl-butyrylamino} acétique, autrement nommé N-[N-acétyl, N'-(3-trifluorométhyl)phényl valyl]glycine ou N-acetyl-N-[3-(trifluoromethyl)phenyl]valyl-glycine ou acetyl trifluoromethyl phenyl valylglycine, ou un ester de celui-ci avec un alcool en $C_1$-$C_6$. ; un extrait de peptides de riz tel que la Colhibin® de Pentapharm, ou un extrait de Phyllanthus emblica tel que l'Emblica® de Rona.

- soit sur la synthèse des glycosaminoglycannes, tels que le produit de fermentation du lait par lactobacillus vulgaris, commercialisé par la société BROOKS sous la dénomination commerciale Biomin yogourth® ; l'extrait d'algue brune Padina pavonica commercialisé par la société ALBAN MÜLLER sous la dénomination commerciale HSP3® ; l'extrait de *Saccharomyces cerevisiae* disponible notamment auprès de la société SILAB sous la dénomination commerciale Firmalift® ou auprès de la société LSN sous la dénomination commerciale Cytovitin® ; un extrait de *Laminaria ochroleuca* tel que la Laminaïne® de Secma ; l'essence de Mamaku de Lucas Meyer, un extrait de cresson (Odraline® de Silab).

- soit sur la synthèse de la fibronectine, tels que l'extrait de zooplancton Salina commercialisé par la société SEPORGA sous la dénomination commerciale GP4G® ; l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil®.

Parmi les actifs stimulant les macromolécules épidermiques, telles que la fillagrine et les kératines, on peut citer notamment l'extrait de lupin commercialisé par la société SILAB sous la dénomination commerciale Structurine® ; l'extrait de bourgeons de hêtre Fagus sylvatica commercialisé par la société GATTEFOSSE sous la dénomination commerciale Gatuline® RC; et l'extrait de zooplancton Salina commercialisé par la société SEPORGA sous la dénomination commerciale GP4G® ; Tripeptide de Cuivre de PROCYTE ; ; un extrait peptidique de *Voandzeia substerranea* tel que celui commercialisé par la société Laboratoires Sérobiologiques sous la dénomination commerciale Filladyn LS

9397®.

De préférence on utilisera un agent additionnel stimulant la synthèse de macromolécules dermiques et/ou épidermiques et/ou empêchant leur dégradation choisi parmi les agents stimulant la synthèse des glycosaminoglycanes, les agents inhibant la dégradation de l'élastine, les agents stimulant la synthèse de la fibronectine, les agents stimulant la synthèse de macromolécules épidermiques, et leurs mélanges.

Encore plus préférentiellement, on utilisera un agent stimulant la synthèse des glycosaminoglycanes choisi parmi un extrait d'algue brune Padina pavonica, un extrait de *Saccharomyces cerevisiae,* un extrait de *Laminaria ochroleuca,* l'essence de Mamaku, un extrait de cresson et leurs mélanges.

Comme agents additionnels préférés stimulant la synthèse de macromolécules dermiques et/ou épidermiques et/ou empêchant leur dégradation, on peut citer : le biopeptide CL ou palmitoyloligopeptide, un extrait d'aphanizomenon flos-aquae (cyanophycée), un extrait de malt, un extrait de moringa, un extrait de *Saccharomyces cerevisiae,* un extrait peptidique de graines de *Pisum sativum,* l'acide {2-[acétyl-(3-trifluorométhyl-phényl)-amino]-3-méthyl-butyrylamino} acétique), un extrait d'algue brune *Padina pavonica,* un extrait de zooplancton Salina, un extrait de levure, un extrait de lupin, un extrait de bourgeons de hêtre Fagus sylvatica, un extrait peptidique de *Voandzeia substerranea,* et leurs mélanges.
Comme actifs préférés stimulant la synthèse de macromolécules dermiques et/ou épidermiques et/ou empêchant leur dégradation, on peut citer :

les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; les peptides de riz tel que le Nutripeptide® de SILAB, le méthylsilanol mannuronate tel que l'Algisium C® commercialisé par Exsymol ;; l'acide folique ; un extrait de Medicago sativa (alfafa) tel que celui commercialisé par SILBA sous la dénomination Vitanol®; un extrait peptidique de noisette tel que celui commercialisé par la société Solabia sous la dénomination Nuteline C®; l'arginine ; . un extrait d'aphanizomenon flos-aquae (cyanophycée) commercialisée sous la dénomination Lanablue® par Atrium Biotechnologies, l'extrait de malt commercialisé par la société COLETICA sous la dénomination commerciale Collalift® le lycopène ; un extrait de litchi ; un extrait de moringa tel que l'Arganyl LS 9781® de Cognis ; un extrait de vaccinium myrtillus tel que ceux décrits dans la demande FR-A-2814950 ; : le retinol et dérivés en particulier le palmitate de rétinol ; l'extrait de Saccharomyces Cerivisiae commercialisé par la société LSN sous la dénomination commerciale Cytovitin® ; un extrait peptidique de noisette tel que celui commercialisé par la société Solabia sous la dénomination Nuteline C®; l'acide {2-[acétyl-(3-trifluorométhyl-phényl)-amino]-3-méthyl-butyrylamino} acétique, autrement nommé N-[N-acétyl, N'-(3-trifluorométhyl)phényl valyl]glycine ou N-acetyl-N-[3-(trifluoromethyl)phenyl]valyl-glycine ou acetyl trifluoromethyl phenyl valylglycine, ou un ester de celui-ci avec un alcool en $C_1$-$C_6$ ; un extrait de peptides de riz tel que la Colhibin® de Pentapharm, ou un extrait de Phyllanthus emblica tel que l'Emblica® de Rona ; l'extrait d'algue brune Padina pavonica commercialisé par la société ALBAN MÜLLER sous la dénomination commerciale HSP3® ; l'extrait de Saccharomyces cerevisiae disponible notamment auprès de la société SILAB sous la dénomination commerciale Firmalift® ou auprès de la société LSN sous la dénomination commerciale Cytovitin® ; un extrait de Laminaria ochroleuca tel que la Laminaïne® de Secma ; l'essence de Mamaku de Lucas Meyer, l'extrait de lupin commercialisé par la société SILAB sous la dénomination commerciale Structurine® ; l'extrait de bourgeons de hêtre Fagus sylvatica commercialisé par la société GATTEFOSSE sous la dénomination commerciale Gatuline® RC.

3. Agents stimulant la prolifération des fibroblastes ou des kératinocytes et/ou la différenciation des kératinocytes

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; un extrait de protéines hydrolysées de soja tel que le RIDULISSE® de SILAB ; et les hormones végétales telles que les giberrellines et les cytokinines ; un extrait peptidique de noisette tel que celui commercialisé par la société Solabia sous la dénomination Nuteline C® et les hormones végétales telles que les giberrellines et les cytokinines.

De préférence on utilisera un agent favorisant la prolifération et/ou la différenciation des kératinocytes.

Les agents stimulant la prolifération des kératinocytes, utilisables dans la composition selon l'invention, comprennent notamment l'adénosine ; le phloroglucinol ; les extraits de tourteaux de noix commercialisés par la société GATTEFOSSE ; et les extraits de Solanum tuberosum tels que le Dermolectine® commercialisé par la société SEDERMA ; un extrait de Larrea divaricata tel que le Capislow® de Sederma, les mélanges de papaye, feuilles d'olivier et citron tel que la Xyléine de Vincience, l'extrait de feuille d'hydrangea macrophylla comme l'Amacha liquid E® d'Ichimaru Pharcos, un extrait de levure tel que le Stimoderm® de CLR.

Parmi les agents stimulant la différenciation des kératinocytes comprennent par exemple les minéraux tels que

le calcium; un extrait peptidique de lupin tel que celui commercialisé par la société SILAB sous la dénomination commerciale Structurine®; le beta-sitosteryl sulfate de sodium tel que celui commercialisé par la société SEPORGA sous la dénomination commerciale Phytocohésine® ; et un extrait hydrosoluble de maïs tel que celui commercialisé par la société SOLABIA sous la dénomination commerciale Phytovityl® ; un extrait peptidique de *Voandzeia substerranea* tel que celui commercialisé par la société Laboratoires Sérobiologiques sous la dénomination commerciale Filladyn LS 9397® ; et les lignanes tels que le sécoisolaricirésinol, le rétinol et ses esters dont le palmitate de retinyl.

Comme agents stimulant la prolifération et/ou la différenciation des kératinocytes, on peut citer encore les oestrogènes tel que l'oestradiol et homologues ; les cytokines.

Comme agents additionnels stimulant la prolifération des fibroblastes ou des kératinocytes et/ou la différenciation des kératinocytes préférés, on citera un extrait de lupin, un extrait de Larrea divaricata, un extrait de levure et leurs mélanges.

4. Agents favorisant la fonction barrière cutanée

Comme agent ayant un effet restructurant de la barrière cutanée, on peut citer un extrait de *Thermus thermophilus* tel que le Vénucéane® de Sederma, un extrait de rhizome d'igname sauvage (dioscorea villosa) tel que l'Actigen Y® d'Active Organics, des extraits de plancton comme l'omega plancton® de Secma, des extraits de levure comme le Relipidium® de Coletica, un extrait de chataigne tel que la Recoverine® de Silab, un extrait de bourgeon de cèdre tel que le Gatuline Zen® de Gattefossé, des sphingosines comme la salicyloyl sphingosine comme la Phytosphingosine SLC de Degussa, un mélange de xylitol, de xylityl polyglycoside et de xylitan comme l'Aquaxyl® de Seppic, extraits de solanacée comme le Lipidessence® de Coletica, les huiles insaturées en oméga 3 telles que les huiles de rosier muscat et leurs mélanges.

On peut encore citer notamment les céramides et dérivés, en particulier les céramides de type 2 (comme la N-oléoyldihydrosphingosine ), de type 3 (comme la stearoyl-4-hydroxysphinganine en nom INCI) et de type 5 (comme la N-2-hydroxypalmitoyldihydrosphingosine, ayant pour nom INCI :hydroxypalmytoyl sphinganine), les composés à base de sphingoïdes, les glycosphingolipides, les phospholipides, le cholestérol et ses dérivés, les phytostérols, les acides gras essentiels, le diacylglycérol, la 4-chromanone et dérivés de chromon, la vaseline, la lanoline, les beures de karité, le cocoa butter, la lanoline...

Comme agents additionnels préférés favorisant la fonction barrière cutanée, on citera un extrait de *Thermus thermophilus,* un extrait de rhizome d'igname sauvage (dioscorea villosa), un extrait de levure, un extrait de chataigne, un extrait de bourgeon de cèdre, l'arginine, la sérine, les céramides notamment de type 3 et 5 et leurs mélanges.

5. Agents anti-glycation

Comme agents anti-glycation, on peut citer notamment l'ergothionéine, les dihydroxystilbènes et leurs dérivés, les hydroxy imides et dérivés comme ceux cités dans la demande WO2005/054192, l'Antiglyskin® de Silab, les polypeptides d'arginine et de lysine comme l'Amadorine® de Solabia, les extraits de vin tel que l'extrait de vin blanc en poudre sur support maltodextrine vendu sous la dénomination « Vin blanc déshydraté 2F » par la société Givaudan , le Nutralip® de Labochim, un mélange d'extrait de busserole et de glycogène marin comme l'Aglycal LS 8777® de LSN, un extrait de thé noir comme le Kombuchka® de Sederma., l'acide lipoique ou thioctique, ou des extraits végétaux de la famille des Ericaceae tels que les extraits de myrtille *(Vaccinium myrtillus),* par exemple celui vendu sous la dénomination « BLUEBERRY HERBASOL EXTRACT PG » par la société COSMETOCHEM, le chorhydrate de carcinine (commercialisé par Exsymol sous la dénomination « ALISTIN®»), un extrait d'Hélianthus annuus comme l'Antiglyskin® de Silab, l'acide thioctique (ou acide alpha lipoïque), et leurs mélanges

Comme agents anti-glycation préférés, on citera les extraits de myrtille *(Vaccinium myrtillus).*

6. Agents favorisant la maturation de l'enveloppe cornée

On pourra utiliser dans les compositions de l'invention des agents qui interviennent sur la maturation de l'enveloppe cornée qui s'altère avec l'âge et induit une diminution de l'activité des transglutaminases. On peut citer par exemple l'urée et ses dérivés et en particulier l'Hydrovance® de National Starch et les autres actifs mentionnés dans la demande L'OREAL FR2877220 (non publiée).

7. Inhibiteurs de NO-synthases

On peut également associer des agents ayant une action d'inhibiteur de NO synthase comme les OPC (oligomères procyanidoliques), les extraits de végétal de l'espèce Vitis vinifera notamment commercialisés par la société Euromed sous la dénomination Leucocyanidines de raisins extra, ou encore par la société Indena sous la dénomination

Leucoselect®, ou enfin par la société Hansen sous la dénomination Extrait de marc de raisin, des extraits d'Olea europea de préférence obtenus à partir de feuilles d'olivier tels que ceux de Vinyals sous forme d'extrait sec, ou par la société Biologia & Technologia sous la dénomination commerciale Eurol® BT, des extraits de gingko biloba (Eurol BT de biologiax technologia) de préférence un extrait aqueux sec de ce végétal vendu par la société Beaufour sous le nom commercial Ginkgo biloba extrait standard, des extraits de pépins de raisins, d'aubépine comme ceux de la société Berkem, d'écorce de pin comme ceux de la société Euromed.

8 Antagonistes des récepteurs périphériques des benzodiazépines (PBR)

On peut citer par exemple le 1-(2-chlorophenyl)-N-(1-methyl-propyl)-3-isoquinoline carboxamide ; les composés décrits dans les demandes WO03/030937, WO03/068753, dérivés de pyridazino[4, 5-b] indole-1-acétamide de formule générale (VII) tels que décrits dans le document WO00/44384.

9. Agents augmentant l'activité de la glande sébacée

On peut citer par exemple le dehydrojasmonate de méthyle, l'hécogénine, l'hédione, le le o-linoleyl-6D-glucose et leurs mélanges.

10. Agents dermorelaxants ou dermodécontractants

On peut citer comme exemples le gluconate de manganèse et autres sels, l'alvérine citrate et ses sels, la glycine, un extrait d'Iris pallida, un hexapeptide (Argériline R de Lipotec) ou les sapogénines comme le Wild yam et les amines carbonylées décrites dans la demande EP1484052. Comme exemple de sapogénines on peut citer celles décrites dans la demande de brevet WO02/47650, en particulier le Wild yam, la diosgénine extrait notamment de Dioscorea opposita ou tout extrait refermant naturellement ou après traitement une ou plusieurs sapogénines (rhizome d'igname sauvage, feuille d'agave qui contient de l'hécogénine et la tigogénine, extrait de liliacées et plus particulièrement le Yacca ou le smilax contenant la smilagéine et la sarsapogénine, ou la racine de salsepareille) ou l'Actigen Y de la société Actives Organics.
On peut également citer le DMAE (diméthyl MEA), les extraits de criste marine, de ciste de Montpellier, d'hélicryse, d'anis, de Para cress, un extrait d'Acmella Oleracea comme la Gatuline expression de Gattefossé.
Comme agents additionnels dermorelaxants préférés, on citera le gluconate de manganèse, la glycine et l'alvérine.

11. Agents favorisant la microcirculation cutanée

On peut associer dans les compositions de l'invention des agents agissant sur la microcirculation cutanée afin d'éviter le ternissement du teint comme par exemple le Kombuchka de Sederma, le Pycnogénol, le gluconate de manganèse (Givobio GMn de Seppic), la Visnadine d'Indena, un extrait de lupin (Eclaline de Silab), l'Epaline 100 des Laboratoires carilène, un extrait de fleur de bigarade (Remoduline de ilab), la vitamine P et ses dérivés comme le Permethol de Sochibios et autres extraits (de ruscus, de marron d'inde, lierre, ginseng, mélilot...) et aussi la caféine, le nicotinate et dérivés, la lysine et dérivés (comme l'Asparlyne de Solabia)...
Comme agents additionnels préférés favorisant la microcirculation cutanée, on citera le Kombuchka, le gluconate de manganèse et la vitamine P et ses dérivés.

12. Agents stimulant le métabolisme énergétique des cellules

On peut associer également des actifs stimulant le métabolisme énérgétique qui se trouve ralentit lors du vieillissement, parmi lesquels on peut citer la biotine, un extrait de Saccharomyces cerevisaie tel que le Phosphovital® de Sederma, le mélange de sels de sodium, de manganèse, de zinc et de magnésium d'acide pyrrolidone carboxylique comme le Physiogenyl® de Solabia, un mélange de gluconate de zinc, de cuivre et de magnésium tel que le Sepitonic M3® de Seppic un beta-glucan issu de Saccharomyces cerevisiae tel que celui commercialisé par la société Mibelle AG Biochemistry ; et leurs mélanges.

13. Agents tenseurs

Par « agent tenseur » utilisable dans les compositions de l'invention, on entend des composés susceptibles d'avoir un effet tenseur, c'est-à-dire pouvant tendre la peau.
De manière générale on entend par agent tenseur selon l'invention tous composés solubles ou dispersibles dans l'eau

à une température allant de 25°C à 50°C à la concentration de 7% en poids dans l'eau ou à la concentration maximale à laquelle ils forment un milieu d'apparence homogène et produisant à cette concentration de 7% ou à cette concentration maximale dans l'eau une rétractation de plus de 15 % dans le test décrit ci-après.
La concentration maximale à laquelle ils forment un milieu d'apparence homogène est déterminée à $\pm$ 10% près et de préférence à $\pm$ 5% près.
On entend par 'milieu d'apparence homogène' un milieu ne présentant pas d'agrégats visibles à l'oeil nu.
Pour la détermination de ladite concentration maximale, l'agent tenseur est ajouté progressivement dans l'eau sous agitation à la défloculeuse à une température allant de 25°C à 50°C, puis le mélange est maintenu sous agitation pendant une heure. On observe ensuite après 24 heures si le mélange ainsi préparé est d'apparence homogène (absence d'agrégats visibles à l'oeil nu).
L'effet tenseur peut être caractérisé par un test *in vitro* de rétraction.
On prépare préalablement et tel que décrit précédemment un mélange homogène de l'agent tenseur dans l'eau, à la concentration de 7% en poids ou à la concentration maximale définie précédemment.
30$\mu$l du mélange homogène est déposé sur une éprouvette rectangulaire (10x40mm , donc présentant une largeur initiale $L_0$ de 10 mm) d'élastomère ayant un module d'élasticité de 20 MPa et une épaisseur de 100$\mu$m.
Après 3h de séchage à 22$\pm$3°C et 40$\pm$10% d'humidité relative HR, l'éprouvette d'élastomère présente une largeur rétractatée, notée $L_{3h}$ due à la tension exercée par l'agent tenseur déposé.
L'effet tenseur (ET) peut alors se quantifier de la façon suivante :

$$ET' = (L_0 - L_{3h} / L_0)x100 \text{ en } \%$$

avec $L_o$ = largeur initiale 10mm
et $L_{3h}$ = largeur après 3h de séchage
L'agent tenseur peut être choisi parmi :

a) les protéines végétales ou animales et leurs hydrolysats ;
b) les polysaccharides d'origine naturelle ;
c) les silicates mixtes ;
d) les particules colloïdales de charges inorganiques ;
e) les polymères synthétiques ;
et les mélanges de ceux-ci.

On peut citer notamment :

(1) les polymères synthétiques, tels que les latex de polyuréthanne ou les latex acrylique-silicone, en particulier ceux décrits dans la demande de brevet EP-1038519, tels qu'un polydiméthyl siloxane greffé propylthio(polyacrylate de méthyle), propylthio(polyméthacrylate de méthyle) et propylthio(polyacide méthacrylique), ou encore un polydiméthyl siloxane greffé propylthio(polyméthacrylate d'isobutyle) et propylthio(polyacide méthacrylique). De tels polymères siliconés greffés sont notamment vendus par la Société 3M sous les dénominations commerciales VS 80, VS 70 ou LO21,
(2) les polymères d'origine naturelle, notamment (a) les polyholosides, par exemple (i) sous forme d'amidon issu notamment de riz, de maïs, de pomme de terre, de manioc, de pois, de froment, d'avoine, etc... ou (ii) sous forme de carraghénanes, alginates, agars, gellanes, polymères cellulosiques et pectines, avantageusement en dispersion aqueuse de microparticules de gel, et (b) les latex constitués par la résine shellac, la gomme de sandaraque, les dammars, les élémis, les copals, les dérivés cellulosiques, et leurs mélanges,
(3) les protéines et hydrolysats de protéines végétales, en particulier de maïs, de seigle, de froment, de sarrasin, de sésame, d'épautre, de pois, de fève, de lentille, de soja et de lupin,
(3) les silicates mixtes, notamment les phyllosilicates et en particulier les Laponites,
(4) les microparticules de cire, choisies par exemple parmi les cires de Carnauba, de Candelila ou d'Alfa,
(5) les particules colloïdales de charge inorganique ayant un diamètre moyen en nombre compris entre 0,1 et 100 nm, de préférence entre 3 et 30 nm, et choisies par exemple parmi : la silice, les composites silice-alumine, l'oxyde de cérium, l'oxyde de zirconium, l'alumine, le carbonate de calcium, le sulfate de baryum, le sulfate de calcium, l'oxyde de zinc et le dioxyde de titane. Comme particules colloïdales composites silice-alumine utilisables dans les compositions selon l'invention, on peut citer par exemple celles commercialisées par la société Grace sous les noms de Ludox AM, Ludox AM-X 6021, Ludox HSA et Ludox TMA.

Comme autre agent additionnel anti-âge, on peut citer la DHEA et ses dérivés, l'adénosine et ses dérivés notamment l'adénosine monophosphate disodique, l'acide boswellique, les extraits de romarin, les caroténoides (B carotène, zéaxanthine, luéine, l'acide cystéique, l'acide n-octanoyl 5-salicylique, l'acide gentisique et ses dérivés, les lignanes et ses dérivés glycosyles, les flavonoides et flavostérones, les dérivés de cuivre, l'acide jasmonique, le resvératrol et ses dérivés, le rétinol et ses dérivés.

Le ou les actifs et/ou ingrédients additionnels utilisés dans la composition selon l'invention peuvent représenter de 0,0001 à 20%, de préférence de 0,01 à 10% et mieux, de 0,01 à 1% en poids para rapport au poids total de la composition.

## Ensemble cosmétique

Selon un autre aspect, l'invention concerne également un ensemble cosmétique comprenant :

i) un récipient délimitant au moins un compartiment, ledit récipient étant fermé par un élément de fermeture ; et
ii) une composition telle que décrite précédemment et disposée à l'intérieur dudit compartiment.

Le récipient peut être sous toute forme adéquate. Il peut être notamment sous forme d'un flacon, d'un tube, d'un pot, d'un étui, d'une boite, d'un sachet ou d'un boîtier.

L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.

Le récipient peut être associé à un applicateur. L'applicateur peut être sous forme d'un pinceau, tel que décrit par exemple dans le brevet FR 2 722 380. L'applicateur peut être sous forme d'un bloc de mousse ou d'élastomère, d'un feutre, ou d'une spatule. L'applicateur peut être libre (houppette ou éponge) ou solidaire d'une tige portée par l'élément de fermeture, tel que décrit par exemple dans le brevet US 5 492 426. L'applicateur peut être solidaire du récipient, tel que décrit par exemple le brevet FR 2 761 959.

Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage. Alternativement, le couplage entre l'élément de fermeture et le récipient se fait autrement que par vissage, notamment via un mécanisme à baïonnette, par encliquetage, serrage, soudage, collage, ou par attraction magnétique. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.
Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube.

Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient.

Le récipient peut être constitué d'un boîtier avec un fond délimitant au moins un logement contenant la composition, et un couvercle, notamment articulé sur le fond, et apte à recouvrir au moins en partie ledit fond. Un tel boîtier est décrit par exemple dans la demande WO 03/018423 ou dans le brevet FR 2 791 042.

Le récipient peut être équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618.

L'invention concerne également un procédé cosmétique de soin et/ou de maquillage de la peau et de ses phanères comprenant l'application sur la peau ou ses phanères d'une composition telle que définie précédemment.

Par 'phanères', on entend selon l'invention la peau, les ongles, les cils et/ou les poils, et les cheveux.
De préférence, il s'agira d'une composition pour la peau.

En particulier le procédé selon l'invention vise favoriser le blanchiement et/ou la dépigmentation de la peau et/ou homogénéiser le teint.

Selon un autre mode de réalisation, il vise à atténuer les imperfections cutanées des peaux grasses, en particulier à matifier la peau.

La brillance de la peau est un problème affectant plus particulièrement les adolescents mais qui peut aussi se manifester à l'âge adulte sous l'effet notamment d'une hyperproduction d'androgènes. Une peau brillante ou grasse est généralement une peau hyper-séborrhéique caractérisée par une sécrétion et une excrétion exagérées de sébum conduisant généralement à un taux de sébum supérieur à 200 $\mu g/cm^2$ mesuré au niveau du front.

Par 'imperfections cutanées des peaux grasses', on entend notamment selon l'invention les désordres esthétiques tels qu'une peau luisante, une moins bonne tenue du maquillage, un grain de peau épais généralement associé à un défaut de desquamation, une peau dont les orifices folliculaires sont dilatés ou comblés de minuscules spicules cornés, voire par des comédons ou points noirs (résultant cependant davantage d'un phénomène de rétention que d'une augmentation de l'excrétion).

Par "matifier", on entend rendre la peau visiblement plus mate, moins brillante. L'effet matifiant de la composition peut notamment être évalué à l'aide d'un gonioréflectomètre, en mesurant le rapport R entre la réflexion spéculaire et la réflexion diffuse. Une valeur de R inférieure ou égale à 2 traduit généralement un effet matifiant.

En particulier, la composition est appliquée sur les zones du visage ou du front présentant une brillance de la peau.

Selon une autre application de l'invention, le procédé vise à atténuer les imperfections cutanées liées au vieillissement, en particulier au vieillissement actinique.

Par 'imperfections cutanées liées au vieillissement cutané', en particulier au vieillissement actinique, on entend notamment une perte de fermeté et/ou d'élasticité et/ou de tonicité et/ou de souplesse de la peau, la formation des rides et des ridules, un aspect terne du teint, l'apparition d'un brunissement et/ou d'un jaunissement de la peau, et/ou l'apparition de tâches de sénescence ou 'tâches de vieillesse'.

Il sera notamment destiné aux personnes à peau mature, voire très mature.

Par 'peaux matures' selon l'invention, on entend notamment des personnes ayant au moins 40 ans.

Par 'peaux très matures' selon l'invention, on entend notamment personnes ayant au moins 50 ans, en particulier au moins 60 ans, voire 65 ans.

Selon un mode particulier de l'invention, le procédé est destiné au soin des personnes ayant une peau de phototype III à VI (peaux foncées).

L'invention concerne encore l'utilisation de l'association (a) d'au moins un dérivé diphényl-méthane hydroxylé de formule (I) ou (II) et (b) d'au moins un ingrédient favorisant la solubilisation dudit dérivé, pour la préparation d'une composition pharmaceutique destinée à traiter les désordres dermatologiques liés à la prolifération de microorganismes sur la peau.

Comme désordres dermatologiques, on peut citer notamment les pelades, les lésions acnéiques, les mycoses, et leur mélange.

De préférence, la composition pharmaceutique sera destinée à traiter les lésions acnéiques, et en particulier les désordres hyperpigmentaires desdites lésions acnéiques.

L'invention sera maintenant illustrée par les exemples non limitatifs suivants.

## EXEMPLES

Exemple 1 : Tests de solubilisation d'un dérivé diphényl méthane hydroxylé de formule (I)

*Protocole :*

On utilise comme dérivé diphényl méthane hydroxylé de formule (I) le composé Phenylethylbenzendiol commercialisé sous la dénomination Symwhite® par Symrise.

Ce composé est pesé et placé dans un pilulier hermétique.

Plusieurs solubilisants sont testés : on place dans un bécher 10ml de solubilisant et on ajoute mg par mg le composé Phenylethylbenzendiol. La suspension, éventuellement portée à 45°C, est agitée par agitation magnétique pendant une heure.

La dissolution ou la non dissolution du dérivé diphényl méthane hydroxylé de formule (I), ainsi que son évolution dans le temps sont ensuite suivies.

La non solubilité du dérivé diphényl méthane hydroxylé de formule (I) dans le solubilisant se caractérise macroscopiquement par un précipité ou juste une solution trouble, et microscopiquement par la présence de cristaux.

*Résultats :*

Les résultats de solubilisation sont présentés dans le tableau suivant :

| Solvants | % max de solubilité du dérivé diphényl méthane hydroxylé de formule (I) |
|---|---|
| dicaprylyl carbonate (CETIOL CC de COGNIS) | 26,74% |
| octyl-2-dodécanol ( EUTANOL G de COGNIS) | 22,52% |
| octylmethoxycinnamate (PARSOL MCX de GIVAUDAN (DSM Nutritional Products) | 26,00% |
| N-lauroylsarcosinate d'isopropyle (ELDEW SL 205 de AJINOMOTO) | 42,00% |
| Dimethyl isosorbide (ARLASOLVE DMI de UNIQEMA) | 31,30% |
| Huile d'abricot (APRICOT KERNEL OIL de DESERT WHALE) | 10,00% |
| Isoparaffine hydrogénée (PARLEAM de NOF CORPORATION) | Pas soluble |

Ce test montre que le solubilisant le plus efficace est le N-lauroylsarcosinate d'isopropyle, qui a permis de solubiliser jusqu'à 42 % en poids de composé diphényl méthane hydroxylé (I) (le reste de la solution étant constituée par le solubilisant).
Le dicaprylyl carbonate, l'octyl-2-dodecanol, l'octylmethoxycinnamate et le dimethylisosorbide sont également de bons solubilisants puisqu'ils ont permis de solubiliser plus de 20% en poids dudit composé diphényl méthane hydroxylé (I).
A titre de comparaison, l'huile d'abricot ne permet de solubiliser au plus 10% dudit composé diphényl méthane hydroxylé (I). Et l'huile de paraffine ne permet pas de solubiliser ce dérivé diphényl méthane hydroxylé (I).

Exemple 2 : Exemples de formulations

Les ingrédients sont donnés en nom INCI.

Exemple 2a) :

Gel pour le corps :

| Nom INCI | % |
|---|---|
| Xanthan gum | 0.4 |
| Potassium hydroxyde | 0.3 |
| Dimethicone PEG phosphate | 2 |
| Phenylethylbenzendiol (Symwhite® de Symrise) | 0.5 |
| Dicaprylyl carbonate | 3.00 |
| cyclohexasiloxane | 10 |
| Alcohol denat. | 15 |
| Water | 67.70 |
| Carbomer | 0.4 |
| Acrylates/C10-30 alkyl acrylate crosspolymer | 0.25 |
| total | 100 |

Exemple n°2b):

Soin du visage, émulsion inverse silicone

| Nom INCI | % |
|---|---|
| Disodium EDTA | 0.05 |
| Dimethicone/vinyl dimethicone crosspolymer | 1.2 |
| Nylon-12 | 5 |
| Phenylethylbenzendiol (Symwhite® de Symrise) | 1 |
| Lauroylsarcosinate d'isopropyl | 3.00 |
| Cyclopentasiloxane | 17.3 |
| Glycerin | 23 |
| Propylene glycol | 6 |
| Dimethicone | 3.8 |
| PEG/PPG-18/18 dimethicone | 2.4 |
| Prunus Armenia (apricot) kernet oil | 3 |
| Phenyl trimethicone | 4 |
| Silica | 3 |
| Water | 27.15 |
| Propylparaben | 0.1 |
| methylparaben | 0.2 |
| total | 100 |

Exemple n°2c) :

Soin du visage et des mains

| Nom INCI | % |
|---|---|
| Myristyl alcohol | 0.025 |
| Stearyl alcohol | 1.025 |
| Phenoxyethanol | 0.8 |
| Potassium hydroxyde | 0.5 |
| Glyceryl stearate | 1.25 |
| Cetyl alcohol | 0.95 |
| Phenylethylbenzendiol (Symwhite® de Symrise) | 1 |
| Dimethyl isosorbide | 4.00 |
| Acide ellagique | 0.5 |
| Cyclopentasiloxane | 15 |
| Glycerin | 5 |
| Hydrogenated polyisobutene | 5 |
| Water | 62.10 |
| Carbomer | 0.6 |
| PEG-50 stearate | 2.5 |

(suite)

| Nom INCI | % |
|---|---|
| PEG-100 stearate | 1.25 |
| total | 100 |

## Revendications

1. Composition comprenant, dans un milieu physiologiquement acceptable, (a) au moins un dérive diphényl-méthane hydroxylé de formule (I)

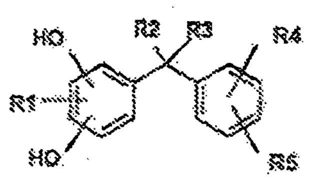

dans laquelle :

- R1 est choisi parmi un atome d'hydrogène, un groupement méthyle, une chaîne alkyle linéaire ou ramifiée, saturée ou insaturée, ayant de 2 à 4 atomes de carbone, un groupement -OH, et un halogène,
- R2 est choisi parmi un atome d'hydrogène, un groupement méthyle, une chaîne alkyle linéaire ou ramifiée, saturée ou insaturée, ayant de 2 à 5 atomes de carbone,
- R3 est choisi parmi un groupement méthyle ou une chaîne alkyle linéaire ou ramifiée, saturée ou insaturée, ayant de 2 à 5 atomes de carbone,
- R4 et R5 sont indépendamment l'un de l'autre choisi parmi un atome d'hydrogène, un groupement méthyle, une chaîne alkyle linéaire ou ramifiée, saturée ou insaturée, ayant de 2 à 5 atomes de carbone, un groupement -OH ou un halogène,

et (b) au moins un ingrédient favorisant la solubilisation dudit dérivé diphényl méthane hydroxylé de formule (I), ledit ingrédient étant choisi parmi : le N-lauroylsarcosinate d'isopropyle, le dimethyl isosorbide, et le dicaprylyl carbonate.

2. Composition selon la revendication 1, **caractérisée en ce que** le dérivé diphényl-méthane hydroxylé a la formule (II) suivante :

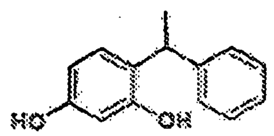

3. Composition selon l'une des revendications 1 ou 2, **caractérisée en ce que** le dérivé diphényl-méthane hydroxylé de formule (I) ou (II) est présent dans la composition en une quantité allant de 0,0001 à 20% en poids par rapport au poids total de la composition, de préférence de 0,001 à 5%, et encore plus préférentiellement de 0,01 à 1% en poids par rapport au poids total de la composition.

4. Composition selon l'une quelconque des revendications 1 ou 2, **caractérisée en ce que** l'ingrédient favorisant la solubilisation desdits dérivés diphényl-méthane de formule (I) ou (II) est le N-lauroylsarcosinate d'isopropyle.

5. Composition selon l'une quelconque des revendications 1 ou 2, **caractérisée en ce que** l'ingrédient favorisant la solubilisation desdits dérivés diphényl-méthane de formule (I) ou (II) est le dimethyl isosorbide.

**6.** Composition selon l'une quelconque des revendication 1 ou 2, **caractérisée en ce que** l'ingrédient favorisant la solubilisation desdits dérivés diphényl-méthane de formule (I) ou (II) est le dicaprylyl carbonate.

**7.** Composition selon l'une quelconque des revendications précédentes, **caractérisée en ce qu'**elle comprend au moins un autre ingrédient et/ou actif additionnel cosmétique ou pharmaceutique choisi parmi les agents dépigmentants, les agents antimicrobiens, les agents antitranspirants, les chélateurs de métaux, les protéines hydrolysées, les antioxydants, les vitamines, les agents anti-inflammatoires, les agents anti-irritants ou apaisants, les agents hydratants, les extraits végétaux, les agents améliorant la fonction barrière, les agents matifiants, les charges abrasives ou agents exfoliants, les agents desquamants, les agents sébo-régulateurs, les agents cicatrisants, les agents astringents, les charges, les azurants optiques, les agents de fluorescence, les agents stimulant la synthèse de macromolécules dermiques et/ou épidermiques et/ou empêchant leur dégradation, les agents stimulant la prolifération des fibroblastes ou des kératinocytes et/ou la différenciation des keratinocytes, les agents favorisant la fonction barrière cutanée, les agents favorisant la maturation de l'enveloppe cornée, les inhibiteurs de NO-synthases, les antagonistes des récepteurs périphériques des benzodiazépines, les agents augmentant l'activité de la glande sébacée, les agents anti-glycation, les agents dermorelaxants ou dermodécontractants, les agents favorisant la microcirculation cutanée, les agents stimulant le métabolisme énergétique des cellules, les agents tenseurs et leurs mélanges.

**8.** Composition selon la revendication 7, **caractérisée en ce que** l'ingrédient et/ou l'actif additionnel représente de 0,0001 à 20% de préférence de 0,01 à 10% et mieux, de 0,01 à 1% en poids par rapport au poids total de la composition.

**9.** Composition selon l'une quelconque des revendications précédentes, **caractérisée par le fait que** la composition est une composition cosmétique de soin de la peau et/ou une composition de maquillage de la peau.

**10.** Composition selon la revendication 9, **caractérisée en ce qu'**il s'agit d'une composition cosmétique de soin et/ou de maquillage de la peau grasse dans laquelle l'ingrédient et/ou l'actif additionnel est choisi parmi agents matifiants, les charges abrasives ou agents exfoliants, les agents desquamants, les agents antimicrobiens, les agents apaisants, les agents anti-inflammatoires, les agents sébo-régulateurs, les agents antioxydants, les agents cicatrisants, les agents astringents, et leurs mélanges.

**11.** Composition selon la revendication 10, **caractérisée en ce qu'**il s'agit d'une composition de soin et/ou de maquillage anti-âge dans laquelle l'ingrédient et/ou actif est choisi parmi les charges, les agents stimulant la synthèse de macromolécules dermiques et/ou épidermiques et/ou empêchant leur dégradation, les agents stimulant la prolifération des fibroblastes ou des kératinocytes et/ou la différenciation des kératinocytes, les agents favorisnt la fonction barrière cutanée, les agents favorisant la maturation de l'enveloppe cornée, les inhibiteurs de NO-synthases, les antagonistes des récepteurs périphériques des benzodiazépines, les agents augmentant l'activité de la glande sébacée, les agents anti-glycation, les agents dermorelaxants ou dermodécontractants, les agents favorisant la microcirculation cutanée, les agents stimulant le métabolisme énergétique des cellules, les agents tenseurs et leurs mélanges.

**12.** Composition selon l'une des revendications 1 à 8, dermatologique pour utilisation dans le traitement de l'acné.

**13.** Ensemble cosmétique ou dermatologique comprenant :

a) un récipient délimitant au moins un compartiment, ledit récipient étant fermé par un élément de fermeture ; et
b) une composition disposée à l'intérieur dudit compartiment, la composition étant conforme à l'une quelconque des revendications qui précèdent.

**14.** Procédé cosmétique de soin et/ou de maquillage de la peau comprenant l'application sur la peau d'une composition telle que définie dans l'une quelconque des revendications 1 à 11.

**15.** Procédé selon la revendication 14, **caractérisé en ce qu'**il vise à favoriser le blanchiement et/ou la dépigmentation de la peau et/ou l'homogénéité du teint.

**16.** Procédé selon la revendication 14, **caractérisé en ce qu'**il vise à atténuer les imperfections cutanées des peaux grasses.

**17.** Procédé selon la revendication 16, **caractérisé en ce que** la composition est appliquée sur les zones du visage ou du front présentant une brillance de la peau.

**18.** Procédé selon la revendication 14, **caractérisée en ce qu'**il vise à atténuer les imperfections cutanées liées au vieillissement cutané.

**19.** Procédé selon la revendication 18,l **caractérisé en ce qu'**il vise à atténuer les imperfections cutanées liées au vieillissement actinique.

**20.** Procédé selon l'une quelconque des revendications 14 à 19, **caractérisé en ce qu'**il est destiné à des personnes ayant une peau de phototype III à VI.

**21.** Utilisation d'au moins (a) un dérivé diphényl-méthane hydroxylé de formule (I) ou (II) tel que défini dans l'une des revendications 1 ou 2 et d'au moins (b) un ingrédient favorisant la solubilisation dudit dérivé, tel que défini dans l'une quelconque des revendications 1 à 6 pour la préparation d'une composition pharmaceutique destinée à prévenir et/ou traiter désordres dermatologiques liés à la prolifération de microorganismes sur la peau.

**22.** Utilisation selon la revendication 21, **caractérisée en ce que** les désordres dermatologiques sont les lésions acnéiques et/ou les désordres hyperpigmentaires desdites lésions acnéiques.

## Patentansprüche

**1.** Zusammensetzung, umfassend in einem physiologisch unbedenklichen Medium (a) mindestens ein hydroxyliertes Diphenylmethanderivat der Formel (I) :

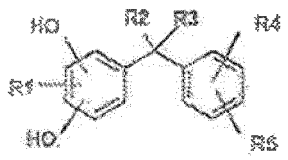

worin:

- R1 aus einem Wasserstoffatom, einer Methylgruppe, einer linearen oder verzweigten, gesättigten oder ungesättigten Alkylkette mit 2 bis 4 Kohlenstoffatomen, einer -OH-Gruppe und einem Halogen ausgewählt ist,
- R2 aus einem Wasserstoffatom, einer Methylgruppe und einer linearen oder verzweigten, gesättigten oder ungesättigten Alkylkette mit 2 bis 5 Kohlenstoffatomen ausgewählt ist,
- R3 aus einer Methylgruppe und einer linearen oder verzweigten, gesättigten oder ungesättigten Alkylkette mit 2 bis 5 Kohlenstoffatomen ausgewählt ist,
- R4 und R5 unabhängig voneinander aus einem Wasserstoffatom, einer Methylgruppe, einer linearen oder verzweigten, gesättigten oder ungesättigten Alkylkette mit 2 bis 5 Kohlenstoffatomen, einer -OH-Gruppe oder einem Halogen ausgewählt sind,

und (b) mindestens einen Bestandteil, der die Solubilisierung des hydroxylierten Diphenylmethanderivats der Formel (I) fördert, wobei der Bestandteil aus Isopropyl-N-lauroylsarcosinat, Dimethylisosorbid und Dicaprylylcarbonat ausgewählt ist.

**2.** Zusammensetzung nach Anspruch 1, **dadurch gekennzeichnet, dass** das hydroxylierte Diphenylmethanderivat die folgende Formel (II) aufweist:

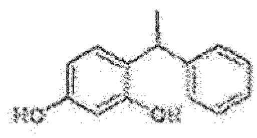

**3.** Zusammensetzung nach Anspruch 1 oder 2, **dadurch gekennzeichnet, dass** das hydroxylierte Diphenylmethanderivat der Formel (I) oder (II) in der Zusammensetzung in einer Menge im Bereich von 0,0001 bis 20 Gew.-%, bezogen auf das Gesamtgewicht der Zusammensetzung, vorzugsweise 0,001 bis 5 Gew.-% und noch weiter bevorzugt 0,01 bis 1 Gew.-%, bezogen auf das Gesamtgewicht der Zusammensetzung, vorliegt.

**4.** Zusammensetzung nach Anspruch 1 oder 2, **dadurch gekennzeichnet, dass** es sich bei dem die Solubilisierung der Diphenylmethanderivate der Formel (I) oder (II) fördernden Bestandteil um Isopropyl-N-lauroylsarcosinat handelt.

**5.** Zusammensetzung nach Anspruch 1 oder 2, I **dadurch gekennzeichnet, dass** es sich bei dem die Solubilisierung der Diphenylmethanderivate der Formel (I) oder (II) fördernden Bestandteil um Dimethylisosorbid handelt.

**6.** Zusammensetzung nach Anspruch 1 oder 2, **dadurch gekennzeichnet, dass** es sich bei dem die Solubilisierung der Diphenylmethanderivate der Formel (I) oder (II) fördernden Bestandteil um Dicaprylylcarbonat handelt.

**7.** Zusammensetzung nach einem der vorhergehenden Ansprüche, **dadurch gekennzeichnet, dass** sie mindestens einen weiteren zusätzlichen kosmetischen oder pharmazeutischen Bestandteil und/oder Wirkstoff umfasst, der aus Depigmentierungsmitteln, antimikrobiellen Mitteln, Antitranspirantien, Metallchelatbildnern, hydrolysierten Proteinen, Antioxidantien, Vitaminen, Antiphlogistika, reizhemmenden oder beruhigenden Mitteln, feuchtigkeitsspendenden Mitteln, Pflanzenextrakten, die Barrierefunktion verbessernden Mitteln, Mattierungsmitteln, abrasiven Füllstoffen oder Exfoliantien, Entschuppungsmitteln, Seborrhoe regulierenden Mitteln, Wundheilungsmitteln, Adstringentien, Füllstoffen, optischen Aufhellern, Fluoreszenzmitteln, Mitteln, die die Synthese von Dermis- und/oder Epidermis-Makromolekülen stimulieren und/oder deren Abbau verhindern, Mitteln, die die Proliferation von Fibroblasten oder Keratinozyten und/oder die Differenzierung von Keratinozyten stimulieren, Mitteln, die die Hautbarrierefunktion fördern, Mitteln, die die Reifung der Hornhülle fördern, NO-Synthase-Inhibitoren, Antagonisten der peripheren Benzodiazepinrezeptoren, Mitteln, die die Aktivität der Talgdrüse erhöhen, Mitteln gegen Glykation, Hautrelaxations- oder Hautdekontraktionsmitteln, Mitteln, die die Hautmikrozirkulation fördern, Mitteln, die den Energiestoffwechsel der Zellen stimulieren, Straffungsmitteln und Mischungen davon ausgewählt ist.

**8.** Zusammensetzung nach Anspruch 7, **dadurch gekennzeichnet, dass** der zusätzliche Bestandteil und/oder Wirkstoff 0,0001 bis 20 Gew.-%, vorzugsweise 0,01 bis 10 Gew.-% und noch besser 0,01 bis 1 Gew.-%, bezogen auf das Gesamtgewicht der Zusammensetzung, ausmacht.

**9.** Zusammensetzung nach einem der vorhergehenden Ansprüche, **dadurch gekennzeichnet, dass** es sich bei der Zusammensetzung um eine kosmetische Zusammensetzung zur Pflege der Haut und/oder eine Zusammensetzung zum Make-up der Haut handelt.

**10.** Zusammensetzung nach Anspruch 9, **dadurch gekennzeichnet, dass** es sich um eine kosmetische Zusammensetzung zur Pflege und/oder zum Make-up von fettiger Haut handelt, wobei der zusätzliche Bestandteil und/oder Wirkstoff aus Mattierungsmitteln, abrasiven Füllstoffen oder Exfoliantien, Entschuppungsmitteln, antimikrobiellen Mitteln, beruhigenden Mitteln, Antiphlogistika, Seborrhoe regulierenden Mitteln, Antioxidantien, Wundheilungsmitteln, Adstringentien und Mischungen davon ausgewählt ist.

**11.** Zusammensetzung nach Anspruch 10, **dadurch gekennzeichnet, dass** es sich um eine der Alterung entgegenwirkende Pflege- und/oder Make-up-Zusammensetzung handelt, wobei der Bestandteil und/oder Wirkstoff aus Füllstoffen, Mitteln, die die Synthese von Dermis- und/oder Epidermis-Makromolekülen stimulieren und/oder deren Abbau verhindern, Mitteln, die die Proliferation von Fibroblasten oder Keratinozyten und/oder die Differenzierung von Keratinozyten stimulieren, Mitteln, die die Hautbarrierefunktion fördern, Mitteln, die die Reifung der Hornhülle fördern, NO-Synthase-Inhibitoren, Antagonisten der peripheren Benzodiazepinrezeptoren, Mitteln, die die Aktivität der Talgdrüse erhöhen, Mitteln gegen Glykation, Hautrelaxations- oder Hautdekontraktionsmitteln, Mitteln, die die Hautmikrozirkulation fördern, Mitteln, die den Energiestoffwechsel der Zellen stimulieren, Straffungsmitteln und Mischungen davon ausgewählt ist.

**12.** Zusammensetzung nach einem der Ansprüche 1 bis 8, dermatologisches, zur Verwendung bei der Behandlung von Akne.

**13.** Kosmetisches oder dermatologisches Set, umfassend:

a) einen Behälter, der mindestens ein Kompartiment begrenzt, wobei der Behälter mit einem Schließelement verschlossen ist; und
b) eine in dem Kompartiment angeordnete Zusammensetzung, wobei die Zusammensetzung einem der vorhergehenden Ansprüche entspricht.

**14.** Kosmetisches Verfahren zur Pflege und/oder zum Make-up der Haut, bei dem man auf die Haut eine Zusammensetzung gemäß einem der Ansprüche 1 bis 11 aufbringt.

**15.** Verfahren nach Anspruch 14, **dadurch gekennzeichnet, dass** es auf die Förderung der Aufhellung und/oder Depigmentierung der Haut und/oder Homogenität des Teints abzielt.

**16.** Verfahren nach Anspruch 14, **dadurch gekennzeichnet, dass** es auf die Abschwächung von Hautunvollkommenheiten fettiger Haut abzielt.

**17.** Verfahren nach Anspruch 16, **dadurch gekennzeichnet, dass** man die Zusammensetzung auf Hautglanz aufweisende Bereiche des Gesichts oder der Stirn aufbringt.

**18.** Verfahren nach Anspruch 14, **dadurch gekennzeichnet, dass** es auf die Abschwächung von mit Hautalterung verbundenen Hautunvollkommenheiten abzielt.

**19.** Verfahren nach Anspruch 18, **dadurch gekennzeichnet, dass** es auf die Abschwächung von mit aktinischer Alterung verbundenen Hautunvollkommenheiten abzielt.

**20.** Verfahren nach einem der Ansprüche 14 bis 19, **dadurch gekennzeichnet, dass** es für Personen mit einer Haut des Phototyps III bis VI bestimmt ist.

**21.** Verwendung von mindestens (a) einem hydroxylierten Diphenylmethanderivat der Formel (I) oder (II) gemäß Anspruch 1 oder 2 und mindestens (b) einem Bestandteil, der die Solubilisierung des Derivats fördert, gemäß einem der Ansprüche 1 bis 6 zur Herstellung einer pharmazeutischen Zusammensetzung zur Prävention und/oder Behandlung von dermatologischen Störungen, die mit der Proliferation von Mikroorganismen auf der Haut verbunden sind.

**22.** Verwendung nach Anspruch 21, **dadurch gekennzeichnet, dass** es sich bei den dermatologischen Störungen um Akne-Läsionen und/oder Hyperpigmentierungsstörungen der Akne-Läsionen handelt.

## Claims

**1.** Composition comprising, in a physiologically acceptable medium, (a) at least one hydroxylated diphenylmethane derivative of formula (I):

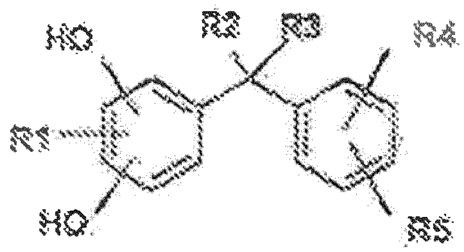

in which:

- R1 is chosen from a hydrogen atom, a methyl group, a saturated or unsaturated and linear or branched alkyl chain having from 2 to 4 carbon atoms, an -OH group or a halogen,
- R2 is chosen from a hydrogen atom, a methyl group or a saturated or unsaturated and linear or branched alkyl chain having from 2 to 5 carbon atoms,

- R3 is chosen from a methyl group or a saturated or unsaturated and linear or branched alkyl chain having from 2 to 5 carbon atoms,
- R4 and R5 are, independently of one another, chosen from a hydrogen atom, a methyl group, a saturated or unsaturated and linear or branched alkyl chain having from 2 to 5 carbon atoms, an -OH group or a halogen,

and (b) at least one ingredient which promotes the dissolution of the said hydroxylated diphenylmethane derivative of formula (I), the said ingredient being chosen from: isopropyl N-lauroylsarcosinate, dimethyl isosorbide and dicaprylyl carbonate.

2. Composition according to Claim 1, **characterized in that** the hydroxylated diphenylmethane derivative has the following formula (II):

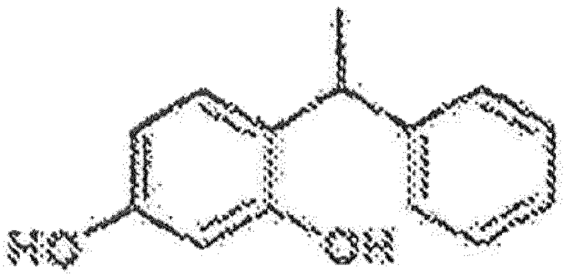

3. Composition according to either of Claims 1 and 2, **characterized in that** the hydroxylated diphenylmethane derivative of formula (I) or (II) is present in the composition in an amount ranging from 0.0001% to 20% by weight, with respect to the total weight of the composition, preferably from 0.001% to 5% by weight and more preferably still from 0.01% to 1% by weight, with respect to the total weight of the composition.

4. Composition according to either one of Claims 1 and 2, **characterized in that** the ingredient which promotes the dissolution of the said diphenylmethane derivatives of formula (I) or (II) is isopropyl N-lauroylsarcosinate.

5. Composition according to either one of Claims 1 and 2, **characterized in that** the ingredient which promotes the dissolution of the said diphenylmethane derivatives of formula (I) or (II) is dimethyl isosorbide.

6. Composition according to either one of Claims 1 and 2, **characterized in that** the ingredient which promotes the dissolution of the said diphenylmethane derivatives of formula (I) or (II) is dicaprylyl carbonate.

7. Composition according to any one of the preceding claims, **characterized in that** it comprises at least one other cosmetic or pharmaceutical additional active agent and/or ingredient chosen from depigmenting agents, antimicrobial agents, antiperspirant agents, metal-chelating agents, hydrolysed proteins, antioxidants, vitamins, anti-inflammatory agents, anti-irritant or soothing agents, moisturizing agents, plant extracts, agents which improve the barrier function, mattifying agents, abrasive fillers or exfoliants, desquamating agents, sebum-regulating agents, healing agents, astringents, fillers, optical brighteners, fluorescence agents, agents which stimulate the synthesis of dermal and/or epidermal macromolecules and/or which prevent their decomposition, agents which stimulate the proliferation of fibroblasts or keratinocytes and/or the differentiation of keratinocytes, agents which promote the skin barrier function, agents which promote the maturing of the horny envelope, NO-synthase inhibitors, peripheral benzodiazepine receptor antagonists, agents which increase the activity of the sebaceous gland, antiglycation agents, dermo-relaxing or dermo-decontracting agents, agents which promote the skin microcirculation, agents which stimulate the energy metabolism of cells, tightening agents and their mixtures.

8. Composition according to Claim 7, **characterized in that** the additional active agent and/or ingredient represents from 0.0001% to 20% by weight, preferably from 0.01% to 10% by weight and better still from 0.01% to 1% by weight, with respect to the total weight of the composition.

9. Composition according to any one of the preceding claims, **characterized in that** the composition is a cosmetic composition for caring for the skin and/or a composition for making up the skin.

**10.** Composition according to Claim 9, **characterized in that** it is a cosmetic composition for caring for and/or making up greasy skin in which the additional activating agent and/or ingredient is chosen from mattifying agents, abrasive fillers or exfoliants, desquamating agents, antimicrobial agents, soothing agents, anti-inflammatory agents, sebum-regulating agents, antioxidants, healing agents, astringents and their mixtures.

**11.** Composition according to Claim 10, **characterized in that** it is an anti-ageing makeup and/or care composition in which the ingredient and/or active agent is chosen from fillers, agents which stimulate the synthesis of dermal and/or epidermal macromolecules and/or which prevent their decomposition, agents which stimulate the proliferation of fibroblasts or keratinocytes and/or the differentiation of keratinocytes, agents which promote the skin barrier function, agents which promote the maturing of the horny envelope, NO-synthase inhibitors, peripheral benzodiazepine receptor antagonists, agents which increase the activity of the sebaceous gland, antiglycation agents, dermo-relaxing or dermo-decontracting agents, agents which promote the skin microcirculation, agents which stimulate the energy metabolism of cells, tightening agents and their mixtures.

**12.** Composition according to one of Claims 1 to 8, dermatological, for use in the treatment of acne.

**13.** Cosmetic or dermatological assembly, comprising:

a) a container delimiting at least one compartment, the said container being closed by a closing member; and
b) a composition placed inside the said compartment, the composition being in accordance with any one of the preceding claims.

**14.** Cosmetic method for caring for and/or making up the skin, comprising the application to the skin of a composition as defined in any one of Claims 1 to 11.

**15.** Method according to Claim 14, **characterized in that** it is targeted at promoting the bleaching and/or the depigmentation of the skin and/or the uniformity of the complexion.

**16.** Method according to Claim 14, **characterized in that** it is targeted at alleviating the skin imperfections of greasy skin.

**17.** Method according to Claim 16, **characterized in that** the composition is applied to the regions of the face or of the forehead exhibiting shininess of the skin.

**18.** Method according to Claim 14, **characterized in that** it is targeted at alleviating the skin imperfections related to skin ageing.

**19.** Method according to Claim 18, **characterized in that** it is targeted at alleviating the skin imperfections related to actinic ageing.

**20.** Method according to any one of Claims 14 to 19, **characterized in that** it is intended for people having skin of phototype III to VI.

**21.** Use of at least (a) one hydroxylated diphenylmethane derivative of formula (I) or (II) as defined in either of Claims 1 and 2 and of at least (b) one ingredient which promotes the dissolution of the said derivative as defined in any one of Claims 1 to 6 in the preparation of a pharmaceutical composition intended to prevent and/or treat dermatological disorders related to the proliferation of microorganisms on the skin.

**22.** Use according to Claim 21, **characterized in that** the dermatological disorders are acne lesions and/or hyperpigmentary disorders of the said acne lesions.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2004105736 A
- EP 1269986 A
- EP 1221933 A, L'OREAL
- EP 0392883 A
- EP 1028120 A
- EP 1555984 A
- FR 8907947
- EP 0661035 A
- EP 0641557 A
- EP 1414390 A
- EP 0274961 A
- EP 0780115 A
- EP 1025901 A
- FR 2787730
- EP 1034839 A
- FR 2659554
- WO 9305753 A
- EP 1637124 A
- EP 0728460 A
- EP 116005 A
- EP 1172077 A
- EP 1353629 A
- EP 1588694 A
- EP 1172089 A
- EP 1466587 A
- JP 2295912 A
- US 5412004 A
- US 5811487 A
- DE 10324567
- EP 1562562 A
- EP 1151742 A, L'OREAL
- FR 2869796
- US 5538793 A
- WO 2005053632 A
- US 3792068 A
- WO 9318743 A
- EP 1529523 A
- EP 1133979 A
- EP 1129694 A
- FR 0108283, L'OREAL
- EP 0852949 A
- EP 0796861 A
- EP 0218200 A
- EP 0899330 A
- EP 1333022 A
- EP 1333021 A
- EP 761204 A
- FR 2812544 A
- FR 2814950 A
- WO 0194381 A
- WO 2005054192 A
- FR 2877220
- WO 03030937 A
- WO 03068753 A
- WO 0044384 A
- EP 1484052 A
- WO 0247650 A
- EP 1038519 A
- FR 2722380
- US 5492426 A
- FR 2761959
- WO 0103538 A
- WO 03018423 A
- FR 2791042
- FR 2792618


### Littérature non-brevet citée dans la description


- *The journal of the American Chemical Society,* Février 1938, vol. 60, 309